(19) Europäisches Patentamt
European Patent Office
Office européen des brevets

(11) **EP 4 039 339 B1**

(12) **EUROPEAN PATENT SPECIFICATION**

(45) Date of publication and mention of the grant of the patent:
**25.06.2025 Bulletin 2025/26**

(21) Application number: **20872898.0**

(22) Date of filing: **29.09.2020**

(51) International Patent Classification (IPC):
***A61Q 17/04*** (2006.01) ***A61K 8/06*** (2006.01)
***A61K 8/31*** (2006.01) ***A61K 8/81*** (2006.01)
***A61K 8/891*** (2006.01) ***A61K 8/898*** (2006.01)

(52) Cooperative Patent Classification (CPC):
**A61Q 17/04; A61K 8/31; A61K 8/891; A61K 8/895; A61K 8/898**

(86) International application number:
**PCT/JP2020/036857**

(87) International publication number:
**WO 2021/065895 (08.04.2021 Gazette 2021/14)**

(54) **SUNSCREEN COSMETIC**

SONNENSCHUTZ-KOSMETIKUM

PRODUIT COSMÉTIQUE D’ÉCRAN SOLAIRE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **30.09.2019 JP 2019180339**

(43) Date of publication of application:
**10.08.2022 Bulletin 2022/32**

(73) Proprietor: **Kao Corporation**
**Tokyo 103-8210 (JP)**

(72) Inventors:
- **MATSUO, Mikano**
  **Chuo-ku, Tokyo 103-8210 (JP)**
- **ARAKAWA, Takashi**
  **Chuo-ku, Tokyo 103-8210 (JP)**

(74) Representative: **Hoffmann Eitle**
**Patent- und Rechtsanwälte PartmbB**
**Arabellastraße 30**
**81925 München (DE)**

(56) References cited:
**EP-A1- 3 804 690**
**WO-A1-2018/221174**
**WO-A1-2019/230274**
**JP-A- 2009 084 171**
**JP-A- 2009 235 007**

- **ANONYMOUS: "To Bee Or Not To Bee | HAPPI", 8 March 2018 (2018-03-08), XP093090217, Retrieved from the Internet <URL:https://www.happi.com/issues/2018-08-01/view_features/to-bee-or-not-to-bee/> [retrieved on 20231010]**
- **DATABASE GNPD [online] Mintel; September 2010 (2010-09-01), "MAX Skin Protect Sunscreen SPF 100", XP055814992, Database accession no. 1380759**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Processed by Luminess, 75001 PARIS (FR)

## Description

### Field of the Invention

The present invention relates to a sunscreen cosmetic.

### Background of the Invention

Sunscreen cosmetics are required not only to have a UV protection effect but also impart a use impression with a user to feel like using repeatedly. For example, an oil-in-water sunscreen cosmetic has been proposed containing a solid or semisolid oil such as an alkyl-modified silicone wax in a specific amount wherein the total amount of oil phases is set below a certain level for obtaining a use impression of being fresh and highly spreadable and an excellent UV protection effect (Patent Literature 1).

However, the sunscreen cosmetic disclosed in Patent Literature 1 has insufficient rub resistance, and is likely to be peeled off when a surface of a part where the sunscreen cosmetic is applied is rubbed.

WO 2018/221174 A1 describes water-in-oil emulsions containing: (a) 0.1-4 mass% of at least one selected from partially crosslinked polyether modified silicone and partially crosslinked polyglycerin modified silicone; (b) 0.05-1.5 mass% of non-crosslinked silicone active agent; (c) 30 mass% or more of an aqueous component; (d) 3.5-20 mass% of an ultraviolet absorbent; and (e) 3-45 mass% of at least one selected from a silicone oil having a viscosity of 1-20 mm2/s at 25°C and an ester oil having an IOB value of 0.1-0.4.

JP 2009-084171 A describes a sunscreen cosmetic that comprises (a) a volatile organopolysiloxane, (b) a silicone-based dispersing agent, (c) a particulate metal oxide surface-treated with a silicone and (d) an organic ultraviolet absorber, wherein the silicone-treated particulate metal oxide (c) is a particulate metal oxide surface-treated with a silicone compound having a structure containing a straight-chain methylpolysiloxane as the main chain and a part of the methyl groups is substituted with a C4-C12 alkyl group, a triethoxysilane group and a methylpolysilxoane group.

The Mintel database contains a record for MAX Skin Protect Sunscreen SPF 100 (Database accession no. 1380759).

JP 2009-235007 A describes and oily cosmetic that comprises: (a) a solid oil, (b) a compound obtained by esterifying with isostearyl alcohol the carboxy groups on both ends of an oligomer ester of a dimer acid and a dimer diol; (c) an ester oil liquid at room temperature 300-1,000 in molecular weight having intramolecular hydroxy groups; and (d) a powder surface-treated with tridecafluorooctyltriethoxysilane.

WO 2019/230274 A1 A describes a skin whitening agent having an aminocarboxylic acid derivative as an active ingredient.

(Patent Literature 1) JP-A-2017-66140
(Patent Literature 2) JP-A-2019-14708

### Summary of the Invention

The present invention provides a sunscreen cosmetic comprising components the following (A), (B) and (C) and which is an oil-in-water emulsion cosmetic:

(A) a wax having a melting point of 55°C or higher in an amount of from 0.05 to 1.5 mass%, wherein the wax is selected the group consisting of a hydrocarbon-based wax and a silicone-based wax, and wherein the silicone-based wax is selected from the group consisting of alkyl methicone wax, alkyl dimethicone wax and silsesquioxane resin wax;
(B) a (meth)acryl-based silicone resin selected from the group consisting of a (meth)acryl-based polymer having a dendrimer-type siloxane structure at a side chain and a (meth)acryl-silicone-based graft copolymer; and
(C) a UV filter in an amount of from 2.5 to 45 mass%, wherein the UV filter is selected from the group consisting of a hydrophobized UV scattering agent and a UV absorber, wherein the hydrophobized UV scattering agent is a hydrophobized particulate metal oxide and the UV absorber is an organic UV absorber selected from the group consisting of a benzoic acid-based UV absorber, an anthranilic acid-based UV absorber, a salicylic acid-based UV absorber, a cinnamic acid-based UV absorber, a benzophenone-based UV absorber, and a triazine-based UV absorber.

### Detailed Description of the Invention

For improving rub resistance when an acryl-based polymer having a dendrimer-type siloxane structure at a side

chain and a UV absorber are used in combination, a sunscreen cosmetic has been proposed containing, in addition to these components, an (acrylates/C10-30 alkyl acrylate) cross-polymer (Patent Literature 2). However, even in such a cosmetic, there remains room for improvement in rub resistance. The cosmetic disclosed in Patent Literature 2 gives a sticky feeling after application, and has an insufficient impression of stay on the skin.

The present invention relates to provision of a sunscreen cosmetic which reliably stays on the skin and has excellent rub resistance.

The present inventors found that by combining a specific amount of a wax having a melting point of 55°C or higher and a (meth)acryl-based silicone resin together with a UV filter, a sunscreen cosmetic can be obtained which reliably stays on the skin and has excellent rub resistance. In this way, the present invention was completed.

The sunscreen cosmetic of the present invention reliably stays on the skin and has excellent rub resistance.

<Component (A)>

The sunscreen cosmetic of the present invention comprises (A) a wax having a melting point of 55°C or higher. The (A) wax conceptually excludes the components (B) and (D).

The melting point of the (A) wax for use in the present invention is 55°C or higher, preferably 57.5°C or higher, more preferably 60°C or higher from the viewpoint of stay on the skin, rub resistance and the like, and preferably 140°C or lower, more preferably 125°C or lower, particularly preferably 110°C or lower from the viewpoint of ease of production and the like. Specifically, the melting point is preferably 55°C or higher and 140°C or lower, more preferably 57.5°C or higher and 125°C or lower, even more preferably 60°C or higher and 110°C or lower.

In the present description, the melting point means a melting point measured in accordance with JIS K 0064.

The (A) wax is preferably an oil-soluble wax. The wax is preferably one that is solid at 25°C at 1 atm.

As long as the melting point of the component (A) is 55°C or higher, the (A) wax is selected the group consisting of hydrocarbon-based waxes and silicone-based waxes. These may be used singly or in combinations of two or more thereof.

Of these, a combination of a hydrocarbon-based wax and a silicone-based wax, or a silicone-based wax is more preferable, and a combination of a hydrocarbon-based wax and a silicone-based wax is particularly preferable, from the viewpoint of stay on the skin, rub resistance and the like. When a hydrocarbon-based wax and a silicone-based wax are used in combination, the mass ratio of (A-2) a silicone-based wax to (A-1) a hydrocarbon-based wax, [(A-2)/(A-1)], is preferably from 0.1 to 10, more preferably from 0.25 to 4, even more preferably from 0.5 to 2, from the viewpoint of rub resistance.

Examples of the (A-1) hydrocarbon-based wax include mineral-based hydrocarbon waxes such as ozokerite and ceresin wax; petroleum-based hydrocarbon waxes such as paraffin wax and microcrystalline wax; and synthetic hydrocarbon waxes such as Fisher Tropsch wax, polyethylene wax, and α-olefins having 28 or more carbon atoms. The number of carbon atoms in the α-olefin is preferably from 30 to 60, more preferably from 30 to 50. Examples of the α-olefin include linear α-olefins and branched α-olefins, and linear α-olefins are preferable.

Among the hydrocarbon-based waxes, from the viewpoint of rub resistance, ceresin wax, paraffin wax, microcrystalline wax and synthetic hydrocarbon wax are preferable, and ceresin wax and synthetic hydrocarbon wax are particularly preferable.

The (A-2) silicone-based wax is an alkyl-modified silicone wax and is selected from the group consisting of alkyl methicone wax, alkyl dimethicone wax and silsesquioxane resin wax.

From the viewpoint of rub resistance and a use impression (no stickiness), the alkyl-modified silicone wax is preferably a silicone wax modified with an alkyl group having 9 to 80 carbon atoms.

The alkyl group may be linear or branched. The number of carbon atoms in the alkyl group is preferably 16 or more, more preferably 20 or more, even more preferably 24 or more, particularly preferably 28 or more from the viewpoint of rub resistance, and preferably 70 or less, more preferably 60 or less, particularly preferably 50 or less from the viewpoint of ease of production, a use impression (no filmy feeling) and the like. The site of substitution in this alkyl group is not limited, and the form of substitution may be any of a single-end type, a double-end type, a side-chain type and the like. In addition, the number of substitutions in the alkyl group is not limited, and there may be one or more substitutions in the molecule. When the alkyl group has two or more substitutions, the two or more alkyl groups may be the same or different. A mixture of silicone waxes modified with different alkyl groups may be used.

The alkyl-modified silicone wax may include C26-28 alkyl methicone and C30-45 alkyl methicone as examples of the alkyl methicone wax; C30-45 alkyl dimethicone wax as an example of the alkyl dimethicone wax; and C30-45 alkyl dimethylsilyl polypropylsilsesquioxane as an example of a silsesquioxane resin wax. These may be used singly or in combinations of two or more thereof. Of these waxes, alkyl methicone waxes and silsesquioxane resin waxes are preferable, and alkyl methicone waxes are particularly preferable, from the viewpoint of stay on the skin, rub resistance and the like.

As the wax having a melting point of 55°C or higher, a commercialized product may be used, or one obtained by

performing synthesis in accordance with a conventional method may be used. Examples of the commercialized product of the ceresin wax include Ceresin #810 (manufactured by NIKKO RICA CORPORATION) and Ceresin Wax SP-1020P (manufactured by Strahl & Pitsch, Inc.). Examples of the commercialized product of the paraffin wax include Paraffin Waxes 140, 145, 150, 155 and HNP-3, 5 and 9 (each manufactured by NIPPON SEIRO CO., LTD.). Examples of the commercialized product of the microcrystalline wax include Multiwaxes W-445 and W-835 (each manufactured by Sonneborn LLC.), Paracera M (manufactured by Paramelt B.V.), Hi-Mic-1045, 1070, 2045 and HNP-0190 (each manufactured by NIPPON SEIRO CO., LTD.), Purified Microcrystalline Wax (manufactured by NIKKO RICA CORPORATION) and 155° Microwax (manufactured by Nippon Oil Corporation). Examples of the commercialized product of the polyethylene wax include PERFORMALENEs 400, 500, 655, 700 EP and PL (each manufactured by New Phase Technologies LLC), LASH WAX P (deodorized product) (manufactured by Japan Natural Products Co., Ltd.) and Polyethylene Wax PW-655N (manufactured by TOSHIKI PIGMENT CO., LTD.). Examples of the commercialized product of the synthetic hydrocarbon wax include LIPWAX A-4 (manufactured by Japan Natural Products Co., Ltd.) in addition to the polyethylene waxes described above.

Examples of the commercialized product of the C26-28 alkyl methicone include BELSIL CM 7026 VP (manufactured by Wacker Asahikasei Silicone Co., Ltd., melting point: 70°C). Examples of the commercialized product of the C30-45 alkyl dimethicone include SF-1642 (manufactured by Momentive Performance Materials Japan Ltd., melting point: 60 to 70°C). Examples of the commercialized product of the C30-45 alkyl dimethylsilyl polypropylsilsesquioxane include SW-8005 C30 Resin Wax (manufactured by Dow Corning Toray Co., Ltd., melting point: 63 to 71°C). Examples of the commercialized product of the mixture of a C30-45 alkyl methicone and a C30-45 olefin include AMS-C30 Cosmetic Wax (manufactured by Dow Corning Toray Co., Ltd., melting point: 73 to 77°C) .

The waxes having a melting point of 55°C or higher may be used singly or in combinations of two or more thereof.

The content of the wax having a melting point of 55° or higher is from 0.05 mass% to 1.5 mass% in the sunscreen cosmetic of the present invention. The content of the wax having a melting point of 55°C or higher in this range serves to improve stay on the skin of the sunscreen cosmetic.

The content of the wax having a melting point of 55°C or higher is preferably 0.075 mass% or more, more preferably 0.1 mass% or more, in the sunscreen cosmetic of the present invention, from the viewpoint of ease of production and the like, and preferably 1.25 mass% or less, more preferably 1 mass% or less, in the sunscreen cosmetic of the present invention, from the viewpoint of ease of production and the like. Specifically, the content of the wax is preferably from 0.075 mass% to 1.25 mass%, more preferably from 0.1 mass% to 1 mass%, in the sunscreen cosmetic of the present invention. The content of the (A-2) silicone-based wax is preferably 0.25 mass% or more, more preferably 0.5 mass% or more, in the sunscreen cosmetic of the present invention, from the viewpoint of stay on the skin, and preferably 1.25 mass% or less, more preferably 1 mass% or less, in the sunscreen cosmetic of the present invention, from the viewpoint of stay on the skin. Specifically, the content of the silicone-based wax is preferably from 0.25 mass% to 1.25 mass%, more preferably from 0.5 mass% to 1 mass%, in the sunscreen cosmetic of the present invention.

<Component (B)>

The sunscreen cosmetic of the present invention comprises (B) a (meth)acryl-based silicone resin selected from the group consisting of a (meth)acryl-based polymer having a dendrimer-type siloxane structure at a side chain and a (meth)acryl-silicone-based graft copolymer. Since the sunscreen cosmetic of the present invention comprises a (meth) acryl-based silicone resin, rub resistance is improved.

In the present description, the (B) (meth)acryl-based silicone resin is one in which a segment containing repetitions of a (meth)acrylate-based monomer-derived structural unit is present at a main chain and a silicone segment is present at a side chain. The silicone segment may have a chain structure or a branched structure such as a tree-like structure.

Examples of the (meth)acryl-based silicone resin include those that are liquid, semisolid or solid at 25°C at 1 atm, and the (meth)acryl-based silicone resin is preferably one that is solid at 25°C at 1 atm. It is also possible to use a (meth) acryl-based silicone resin mixed with a dispersion medium such as decamethylcyclopentasiloxane.

The (meth)acryl-based polymer having a dendrimer-type siloxane structure at a side chain, and (meth)acryl-silicone-based graft copolymer may be used singly or in combinations of two or more thereof.

Among them, from the viewpoint of rub resistance, (meth)acryl-based polymers having a dendrimer-type siloxane structure at a side chain are preferable.

((Meth)acryl-based polymer having dendrimer-type siloxane structure at side chain)

The (meth)acryl-based polymer having a dendrimer-type siloxane structure at a side chain is preferably a (meth) acryl-based polymer having a structural unit of formula (1).

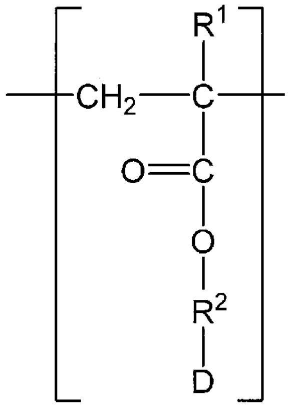

(1)

wherein

$R^1$ represents a hydrogen atom or a methyl group;
$R^2$ represents an alkanediyl group having 1 to 10 carbon atoms; and
D represents a dendrimer-type siloxane structure.

In formula (1), the number of carbon atoms in the alkanediyl group represented by $R^2$ is preferably from 1 to 3. The alkanediyl group represented by $R^2$ may be linear or branched, and examples thereof include a methane-1,1-diyl group, an ethane-1,1-diyl group, an ethane-1,2-diyl group, a propane-1,1-diyl group, a propane-1,2-diyl group, a propane-1,3-diyl group and a propane-2,2-diyl group.

Examples of the dendrimer-type siloxane structure include structures of formula (2).

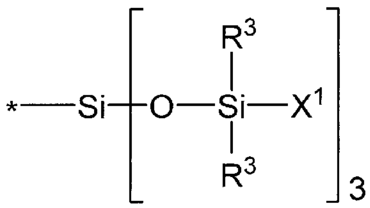

(2)

wherein

$R^3$ represents an alkyl group having 1 to 10 carbon atoms, or an aryl group;
$X^1$ represents a silylalkyl group of formula (3) in which i is 1; and
* represents a bond.

In formula (2), examples of the alkyl group represented by $R^3$ include linear or branched alkyl groups having 1 to 10 carbon atoms (preferably 1 to 5 carbon atoms), such as a methyl group, an ethyl group, a propyl group, an isopropyl group, a butyl group, an isobutyl group and a pentyl group; and cycloalkyl groups having 3 to 10 carbon atoms (preferably 4 to 8 carbon atoms), such as a cyclopentyl group and a cyclohexyl group.

Examples of the aryl group include a phenyl group and a naphthyl group.

$R^3$ is preferably a methyl group or a phenyl group, particularly preferably a methyl group.

A plurality of $R^3$s present in formula (2) may be the same or different. Similarly, a plurality of $X^1$s may be the same or different.

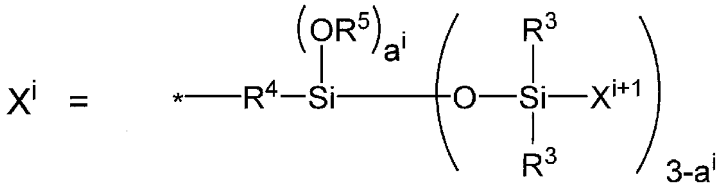

(3)

wherein

$R^3$ has the same meaning as described above;
$R^4$ represents an alkanediyl group having 2 to 10 carbon atoms;
$R^5$ represents an alkyl group having 1 to 10 carbon atoms;
$X^{i+1}$ represents a hydrogen atom, an alkyl group having 1 to 10 carbon atoms, an aryl group, or the same silylalkyl group as $X^i$;
$a^i$ represents an integer of 0 to 3; and
* represents a bond.

The number of carbon atoms in the alkanediyl group represented by $R^4$ is preferably from 2 to 6. The alkanediyl group represented by $R^4$ may be linear or branched, and examples thereof include an ethane-1,1-diyl group, an ethane-1,2-diyl group, a propane-1,1-diyl group, a propane-1,2-diyl group, a propane-1,3-diyl group, a propane-2,2-diyl group, a butane-1,4-diyl group, a pentane-1,5-diyl group, a hexane-1,6-diyl group, a hexane-1,5-diyl group and a hexane-2,5-diyl group. Of these, an ethane-1,2-diyl group is particularly preferable.

The alkyl group represented by $R^5$ may be the same as the alkyl group represented by $R^3$, and is preferably a methyl group, an ethyl group, a propyl group, an isopropyl group or a butyl group.

The alkyl group and the aryl group represented by $X^{i+1}$ are preferably the same as those represented by $R^3$.

When $a^i$ is 2 or 3, two or three $R^5$s may be the same or different. When $a^i$ is 0 or 1, two or three $X^{i+1}$s may be the same or different.

i is an integer of 1 to 10, and means the number of stratums of the silylalkyl group (number of repetitions of the silylalkyl group).

The dendrimer-type siloxane structure is preferably one in which the number of stratums of the silylalkyl group is 1. Such a dendrimer-type siloxane structure can be represented by formula (4).

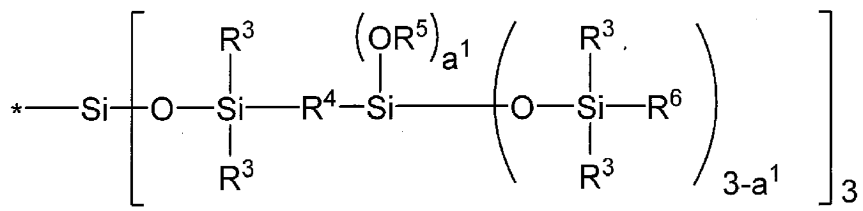

(4)

wherein

$R^6$ represents a hydrogen atom, an alkyl group having 1 to 10 carbon atoms, or an aryl group, where the alkyl group and the aryl group represented by $R^6$ is preferably those represented by $R^3$;
$a^1$ has the same meaning as $a^i$, provided that the average total number of $a^1$s in the dendrimer-type siloxane structures is preferably from 0 to 7; and
other symbols have the same meanings as described above.

The (meth)acryl-based polymer having a dendrimer-type siloxane structure at a side chain is preferably one having, in addition to structural units of formula (1), (meth)acryl-based monomer-derived structural units other than the structural units of formula (1).

Examples of the (meth)acrylate-based monomer include alkyl (meth)acrylates, and glycidyl (meth)acrylates. These may be used singly or in combinations of two or more thereof.

The alkyl group present in the alkyl (meth)acrylate may be linear, branched or cyclic. The alkyl (meth)acrylate is preferably one having an alkyl group having 1 to 30 carbon atoms, more preferably one having an alkyl group having 1 to 22 carbon atoms, more preferably one having an alkyl group having 1 to 8 carbon atoms, even more preferably one having an alkyl group having 1 to 3 carbon atoms, particularly preferably one having an alkyl group having 1 carbon atom. Examples of the alkyl (meth)acrylate include methyl (meth)acrylate, ethyl (meth)acrylate, n-propyl (meth)acrylate, isopropyl (meth) acrylate, n-butyl (meth)acrylate, isobutyl (meth)acrylate, tert-butyl (meth)acrylate, n-hexyl (meth)acrylate, cyclohexyl (meth)acrylate, 2-ethylhexyl (meth)acrylate, octyl (meth)acrylate, lauryl (meth)acrylate, stearyl (meth)acrylate and behenyl (meth)acrylate. Of these, methyl (meth)acrylate, 2-ethylhexyl (meth)acrylate, stearyl (meth)acrylate and behenyl (meth)acrylate are preferable, and methyl (meth)acrylate is particularly preferable.

The total content of structural units of formula (1) and alkyl (meth)acrylate-derived structural units is preferably from 90 mol% to 100 mol%, more preferably from 95 mol% to 100 mol%, particularly preferably from 99 mol% to 100 mol%, with respect to all structural units at the main chain.

As the (meth)acryl-based polymer having a dendrimer-type siloxane structure at a side chain, a commercialized product may be used, or one obtained by performing synthesis in accordance with a method disclosed in JP-A-hei

11-1530, JP-A-2000-63225 or the like may be used. For example, FA 4001 CM (30 mass% decamethylcyclopentasiloxane solution), FA 4002 ID (40 mass% isododecane solution) and FA 4003 DM (40 mass% dimethicone solution) (each manufactured by Dow Corning Toray Co., Ltd.) are on the market as (acrylates/polytrimethylsiloxy methacrylate) copolymers.

((Meth)acryl-silicone-based graft copolymer)

The (meth)acryl-silicone-based graft copolymer is preferably a radical polymer of an organopolysiloxane compound having a radically polymerizable group at an end of a molecular chain and a (meth)acrylate-based monomer other than the organopolysiloxane compound. For example, one disclosed in JP-A-hei 2-25411, JP-A-hei 2-132141, JP-A-hei 3-162442, JP-A-2003-104825 or the like can be used.

The (meth)acryl-silicone-based graft copolymer is preferably one having a structural unit of formula (5).

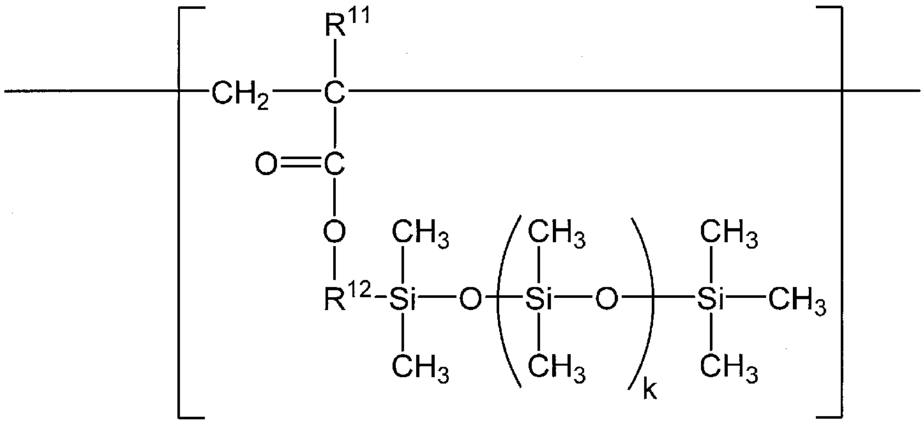

(5)

wherein

$R^{11}$ represents a hydrogen atom or a methyl group;
$R^{12}$ represents an alkanediyl group having 1 to 10 carbon atoms; and
k represents an integer of 3 to 300.

In formula (5), the number of carbon atoms in the alkanediyl group represented by $R^{12}$ is preferably from 1 to 3. The alkanediyl group represented by $R^{12}$ may be linear or branched, and examples thereof include a methane-1,1-diyl group, an ethane-1,1-diyl group, an ethane-1,2-diyl group, a propane-1,1-diyl group, a propane-1,2-diyl group, a propane-1,3-diyl group and a propane-2,2-diyl group.

k is preferably an integer of 5 to 100.

The (meth)acryl-silicone-based graft copolymer is preferably one having, in addition to structural units of formula (5), in addition to structural units of formula (5), (meth)acryl-based monomer-derived structural units other than the structural units of formula (5).

Examples of the (meth)acrylate-based monomer include alkyl (meth)acrylates, and glycidyl (meth)acrylates. These may be used singly or in combinations of two or more thereof.

The alkyl (meth)acrylate is preferably the same as an alkyl (meth)acrylate which may be used in the (meth)acryl-based polymer having a dendrimer-type siloxane structure at a side chain.

The molar ratio of structural units of formula (5) to alkyl (meth)acrylate-derived structural units in the (meth)acryl-silicone-based graft polymer is preferably from 1 : 10 to 10 : 1.

The total content of structural units of formula (5) and alkyl (meth)acrylate-derived structural units is preferably from 90 mol% to 100 mol%, more preferably from 95 mol% to 100 mol%, particularly preferably from 99 mol% to 100 mol%, with respect to all structural units at the main chain.

Specific examples of the (meth)acryl-silicone-based graft copolymer include (acrylates/dimethicone) copolymers, (acrylates/ethylhexyl acrylate/dimethicone methacrylate) copolymers, (acrylates/stearyl acrylate/dimethicone methacrylate) copolymers and (acrylates/behenyl acrylate/dimethicone methacrylate) copolymers (display names for cosmetic products).

As the (meth)acryl-silicone-based graft copolymer, a commercialized product may be used, or one obtained by performing synthesis in accordance with a conventional method may be used. Examples of the commercialized product of the (acrylates/dimethicone) copolymer include KP-541 (mixture with isopropanol, content: 60 mass%), KP-545 (mixture

with decamethylcyclopentasiloxane, content: 30 mass%), KP-549 (mixture with methyl trimethicone, content: 40 mass%) and KP-550 (mixture with isododecane, content: 40 mass%) (each manufactured by Shin-Etsu Chemical Co., Ltd.).

Examples of the commercialized product of the (acrylates/ethylhexyl acrylate/dimethicone methacrylate) include KP-575 (mixture with decamethylcyclopentasiloxane, content: 30 mass%), examples of the commercialized product of the (acrylates/stearyl acrylate/dimethicone methacrylate) copolymer include KP-561P, and examples of the commercialized product of the (acrylates/behenyl acrylate/dimethicone methacrylate) copolymer include KP-562P (each manufactured by Shin-Etsu Chemical Co., Ltd.).

The (meth)acryl-based silicone resins may be used singly or in combinations of two or more thereof.

The content of the (meth)acryl-based silicone resin is preferably 0.01 mass% or more, more preferably 0.05 mass% or more, even more preferably 0.1 mass% or more, particularly preferably 0.2 mass% or more, in the sunscreen cosmetic of the present invention, from the viewpoint of rub resistance and the like, and preferably 10 mass% or less, more preferably 6 mass% or less, even more preferably 3 mass% or less, particularly preferably 1.5 mass% or less, in the sunscreen cosmetic of the present invention, from the viewpoint of rub resistance, stay on the skin and the like. Specifically, the content of the (meth)acryl-based silicone resin is preferably 0.01 mass% or more and 10 mass% or less, more preferably 0.05 mass% or more and 6 mass% or less, further more preferably 0.1 mass% or more and 3 mass% or less, even more preferably 0.1 mass% or more and 1.5 mass% or less, particularly preferably 0.2 mass% or more and 1.5 mass% or less, in the sunscreen cosmetic of the present invention.

The mass ratio of the component (A) to the component (B), [(A)/(B)], is preferably 0.01 or more, more preferably 0.05 or more, further more preferably 0.1 or more, even more preferably 0.4 or more, particularly preferably 0.55 or more, and preferably 10 or less, more preferably 5 or less, even more preferably 2.5 or less, particularly preferably 1.5 or less. Specifically, the mass ratio [(A)/(B)] is preferably 0.01 or more and 10 or less, more preferably 0.05 or more and 5 or less, further more preferably 0.1 or more and 2.5 or less, even more preferably 0.4 or more and 1.5 or less, particularly preferably 0.55 or more and 1.5 or less.

<Component (C)>

The sunscreen cosmetic of the present invention comprises (C) a UV filter.

The UV filter includes one or more selected from the group consisting of a hydrophobized UV scattering agent and a UV absorber, wherein the hydrophobized UV scattering agent is a hydrophobized particulate metal oxide and the UV absorber is an organic UV absorber as described below.

(Hydrophobized UV scattering agent)

The hydrophobized UV scattering agent is a hydrophobized particulate metal oxide.

From the viewpoint of accessibility, the particulate metal oxide used for the hydrophobized particulate metal oxide is preferably one or more particulate metal oxides selected from the group consisting of particulate zinc oxide, particulate titanium oxide, particulate cesium oxide, particulate iron oxide and particulate chromium oxide. Of these particulate metal oxides, one or more particulate metal oxides selected from the group consisting of particulate zinc oxide, particulate titanium oxide and particulate cesium oxide are preferable, one or more particulate metal oxides selected from the group consisting of particulate zinc oxide and particulate titanium oxide are more preferable, and particulate zinc oxide is particularly preferable, from the viewpoint of UV protection effect, rub resistance and the like.

A very small amount of an element with a valence of +2 or more can be incorporated in these particulate metal oxides, and one of metals such as iron, zirconium, calcium, manganese, magnesium and yttrium or a combination of two or more thereof can be incorporated in the particulate metal oxides.

The shape of the "particulate metal oxide" is not particularly limited, and examples thereof include a spherical shape, a plate shape, a rod shape, a spindle shape, a needle shape and an irregular shape.

The average particle diameter of the "particulate metal oxide" is preferably 0.01 $\mu$m or more, more preferably 0.012 $\mu$m or more, particularly preferably 0.015 $\mu$m or more, and preferably 1 $\mu$m or less, more preferably 0.8 $\mu$m or less, particularly preferably 0.5 $\mu$m or less. Specifically, the average particle size is preferably in the range of 0.01 $\mu$m or more and 1 $\mu$m or less, more preferably in the range of 0.012 $\mu$m or more and 0.8 $\mu$m or less, particularly preferably in the range of 0.015 $\mu$m or more and 0.5 $\mu$m or less.

The average particle diameter of the particulate metal oxide means an average particle diameter measured by a laser diffraction/scattering method.

As the particulate zinc oxide, for example, FINEX-25, FINEX-30, FINEX-50, FINEX-75 (manufactured by Sakai Chemical Industry Co., Ltd.), MZ 500 series, MZ 700 series (manufactured by TAYCA CORPORATION), ZnO-350 (manufactured by Sumitomo Osaka Cement Company, Ltd.) and the like are on the market. As the particulate titanium oxide, for example, TTO-55 series, TTO-51 series (manufactured by ISHIHARA SANGYO KAISHA, LTD), JR series and JA series (manufactured by TAYCA CORPORATION) are on the market. As the particulate cerium oxide, for example,

high-purity cerium sold by Nikki Co., Ltd. or SEIMI CHEMICAL CO., LTD. may be used.

The hydrophobization treatment of the particulate metal oxide may be performed using a known treatment agent which is hydrophobized, and examples thereof include fluorine compound treatment, silicone treatment, silicone resin treatment, pendant treatment, silane coupling agent treatment, titanium coupling agent treatment, oil agent treatment, N-acylated lysine treatment, polyacrylic acid treatment, metallic soap treatment, amino acid treatment, inorganic compound treatment, plasma treatment, mechanochemical treatment, silane compound treatment and silazane compound treatment.

Of these treatments, from the viewpoint of dispersion stability and the like, treatment with silicone or a silicone resin, treatment with a silane compound or a silazane compound, and treatment with a metallic soap such as aluminum isostearate are preferable.

Examples of the silicone or silicone resin include methylpolysiloxane, methylphenylpolysiloxane, methylhydrogenpolysiloxane, methylhydrogenpolysiloxane-dimethylpolysiloxane copolymers, octamethylcyclotetrasiloxane, decamethylcyclopentasiloxane, dodecamethylcyclohexasiloxane, octamethyltrisiloxane, tetradecamethylhexasiloxane, dimethylsiloxane-methyl(polyoxyethylene)siloxane-methyl(polyoxypropylene)siloxane copolymers, dimethylsiloxane-methyl(polyoxyethylene)siloxane copolymers, dimethylsiloxane-methyl(polyoxypropylene)siloxane copolymers, dimethylsiloxane-methylcetyloxysiloxane copolymers and dimethylsiloxane-methylstearoxysiloxane copolymers. The methylhydrogenpolysiloxane-dimethylpolysiloxane copolymer is preferably a compound of formula (6).

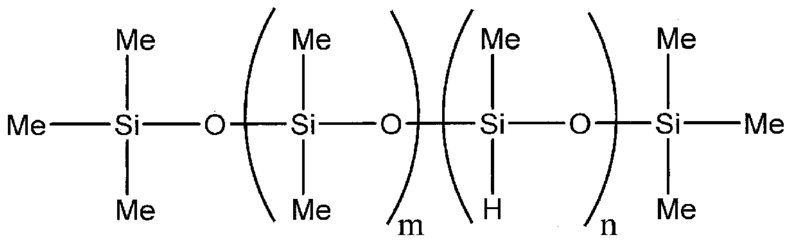

(6)

wherein each of m and n is an integer of 0 or more, where m + n is 1 or more and 60 or less.

The silane compound or silazane compound is preferably a silane compound or silazane compound which has an alkyl group having 1 to 20 carbon atoms or a fluoroalkyl group having 1 to 20 carbon atoms, and is reactive with an inorganic oxide. A silane compound of formula (7) or a silazane compound of formula (8) is preferable. These may be used singly or in combinations of two or more thereof.

$$R^{21}(R^{22})_pSi(Z)_{3-p} \qquad (7)$$

wherein

- $R^{21}$ represents a linear or branched alkyl group having 1 to 20 carbon atoms, or a linear or branched fluoroalkyl group having 1 to 20 carbon atoms;
- $R^{22}$ represents a linear or branched alkyl group having 1 to 6 carbon atoms;
- Z represents a halogen atom or an alkoxy group; and
- p represents 0 or 1.

$$R^{23}R^{24}R^{25}SiNHSiR^{26}R^{27}R^{28} \qquad (8)$$

wherein $R^{23}$ to $R^{28}$ each independently represent a linear or branched alkyl group having 1 to 20 carbon atoms, or a linear or branched fluoroalkyl group having 1 to 20 carbon atoms.

From the viewpoint of dispersion stability and the like, the silane compound is preferably an alkyl alkoxysilane or a fluoroalkyl alkoxysilane, more preferably an alkyl trialkoxysilane or a fluoroalkyl trialkoxysilane. Specific examples thereof include hexyltrimethoxysilane, octyltrimethoxysilane, octyltriethoxysilane, decyltrimethoxysilane, octadecyltrimethoxysilane, trifluoropropyltrimethoxysilane and heptadecafluorodecyltrimethoxysilane. These may be used singly or in combinations of two or more thereof. Of these, octyltrimethoxysilane and octyltriethoxysilane are more preferable.

Specific examples of the silazane compound include hexamethyldisilazane.

The coverage of the surface treatment agent used for hydrophobization treatment is preferably 1 part by mass or more, more preferably 3 parts by mass or more, based on 100 parts by mass of the particulate metal oxide, from the viewpoint of emulsification stability, dispersion stability and the like, and preferably 15 parts by mass or less, more preferably 10 parts by mass or less, based on 100 parts by mass of the particulate metal oxide, from the viewpoint of

emulsification stability, dispersion stability and the like.

As a hydrophobization treatment method, a heretofore known method can be appropriately selected and carried out. For example, for the treatment with silicone or a silicone resin, mention is made of a method in which a particulate metal oxide is covered in a non-vapor state using at least one of silicone compounds including organopolysiloxanes and silicone resins (excluding silane compounds) and then fired in an oxygen-containing atmosphere at a temperature of 600 to 950°C to cover the surface of the particulate metal oxide with silicon oxide as disclosed in JP-B-3187440.

Examples of the treatment method using a silane compound or a silazane compound include chemical bonding methods, and more specific examples include a method in which a silane compound or a silazane compound is mixed with a particulate metal oxide in an organic solvent such as n-hexane, cyclohexane or lower alcohol, the mixture is finely ground if necessary, and the organic solvent is then removed by heating (e.g. 80 to 250°C) or decompression. Mention is also made of a method in which a particulate metal oxide is covered with a polysiloxane compound and then subjected to surface treatment with a silane compound in water as disclosed in JP-A-2007-326902.

The hydrophobized UV scattering agents may be used singly or in combinations of two or more thereof.

(UV absorber)

The UV absorber is an organic UV absorber from the viewpoint of water resistance, rub resistance, solubility and the like.

The organic UV absorber is selected from the group consisting of benzoic acid-based UV absorbers, anthranilic acid-based UV absorbers, salicylic acid-based UV absorbers, cinnamic acid-based UV absorbers, benzophenone-based UV absorbers, and triazine-based UV absorbers. Of these, one or more selected from the group consisting of a cinnamic acid-based UV absorber and a triazine-based UV absorber are preferable from the viewpoint of UV protection effect, solubility and the like. The organic UV absorber is preferably an oil-soluble organic UV absorber.

Examples of the benzoic acid-based UV absorber include para-aminobenzoic acid (PABA), glyceryl PABA, ethyldihydroxypropyl PABA, N-ethoxylate PABA ethyl ester, N-dimethyl PABA ethyl ester, N-dimethyl PABA butyl ester, N-dimethyl PABA amyl ester, octyldimethyl PABA and hexyl diethylaminohydroxybenzoylbenzoate.

Examples of the anthranilic acid UV absorber include homomenthyl-N-acetyl anthranilate.

Examples of the salicylic acid-based UV absorber include amyl salicylate, menthyl salicylate, homomenthyl salicylate, octyl salicylate, phenyl salicylate, benzyl salicylate and p-isopropanol phenyl salicylate.

Examples of the cinnamic acid-based UV absorber include octyl cinnamate, ethyl-4-isopropyl cinnamate, ethyl-2,4-diisopropyl cinnamate, methyl-2,4-diisopropyl cinnamate, propyl-p-methoxycinnamate, isopropyl-p-methoxycinnamate, isoamyl-p-methoxycinnamate, 2-ethylhexyl-p-methoxycinnamate, 2-ethoxyethyl-p-methoxycinnamate, cyclohexyl-p-methoxycinnamate, ethyl-$\alpha$-cyano-$\beta$-phenyl cinnamate, 2-ethylhexyl-$\alpha$-cyano-$\beta$-phenyl cinnamate and glycerylmono-2-ethylhexanoyl diparamethoxycinnamate.

Examples of the benzophenone-based UV absorber include 2,4-dihydroxybenzophenone, 2,2'-dihydroxy-4-methoxybenzophenone, 2,2'-dihydroxy-4,4'-dihydroxybenzophenone, 2-hydroxy-4-methoxybenzophenone, 2-hydroxy-4-methoxy-4'-methylbenzophenone, 2-hydroxy-4-methoxybenzophenone, 4-phenylbenzophenone, 2-ethylhexyl-4'-phenylbenzophenone-2-carboxylate, 2-hydroxy-4-n-octoxybenzophenone and 4-hydroxy-3-carboxybenzophenone.

Examples of the triazine-based UV absorber include 2,4,6-tris[4-(2-ethylhexyloxycarbonyl)anilino]-1,3,5-triazine, dioctylbutamidotriazone and 2,4-bis-[{4-(2-ethylhexyloxy)-2-hydroxy}-phenyl]-6-(4-methoxyphenyl)-1,3,5-triazine.

Examples of other organic UV absorbers include 3-(4'-methylbenzylidene)-dl-camphor, 3-benzylidene-dl-camphor, ethyl urocanate ester, 2-phenyl-5-methylbenzoxazole, 2,2'-hydroxy-5-methylphenylbenzotriazole, 2-(2'-hydroxy-5-t-octylphenyl)benzotriazole, dibenzalazine, dianisoylmethane, 4-methoxy-4'-t-butyldibenzoylmethane, 5-(3,3-dimethyl-2-norbonylidene)-3-pentane-2-one, a benzene-bis-1,3-diketone derivative disclosed in JP-A-2-212579 and a benzoylpinacolone derivative disclosed in JP-A-3-220153.

The organic UV absorbers can be classified broadly into organic UV absorbers which are solid at 25°c at 1 atm and organic UV absorbers which are liquid at 25°C at 1 atm. For example, the above-mentioned hexyl diethylaminohydroxybenzoylbenzoate, 2,4-bis-[{4-(2-ethylhexyloxy)-2-hydroxy}-phenyl]-6-(4-methoxyphenyl)-1,3,5-triazine, 2,4,6-tris[4-(2-ethylhexyloxycarbonyl)anilino]-1,3,5-triazine and the like are organic UV absorbers which are solid at 25°c at 1 atm. The above-mentioned isopropyl-p-methoxycinnamate, 2-ethylhexyl-p-methoxycinnamate and 2-ethoxyethyl-p-methoxycinnamate are organic UV absorbers which are liquid at 25°c at 1 atm.

The UV absorbers may be used singly or in combinations of two or more thereof.

The content of the UV filter is from 2.5 mass% to 45 mass% in the sunscreen cosmetic of the present invention. Since the content of the UV filter is in this range, stickiness after application can be suppressed, and a sufficient UV protection effect can be exhibited.

The content of the UV filter is preferably 5 mass% or more, more preferably 7 mass% or more, particularly preferably 10 mass% or more, in the sunscreen cosmetic of the present invention, from the viewpoint of a UV protection effect, rub resistance and the like, and preferably 40 mass% or less, more preferably 35 mass% or less, particularly

preferably 30 mass% or less, in the sunscreen cosmetic of the present invention, from the viewpoint of no stickiness, storage stability and the like. Specifically, the content of the UV filter is preferably 5 mass% or more and 40 mass% or less, more preferably 7 mass% or more and 35 mass% or less, particularly preferably 10 mass% or more and 30 mass% or less, in the sunscreen cosmetic of the present invention.

When a hydrophobized UV scattering agent is used as the UV filter, the content of the hydrophobized UV scattering agent is preferably 0.5 mass% or more and 25 mass% or less, more preferably 1 mass% or more and 20 mass% or less, particularly preferably 2.5 mass% or more and 15 mass% or less, in the sunscreen cosmetic of the present invention.

When a UV absorber is used as the UV filter, the content of the UV absorber is preferably 0.5 mass% or more and 25 mass% or less, more preferably 1 mass% or more and 20 mass% or less, particularly preferably 2.5 mass% or more and 15 mass% or less, in the sunscreen cosmetic of the present invention.

The mass ratio of the component (A) to the component (C), [(A)/(C)], is preferably 0.0005 or more, more preferably 0.001 or more, from the viewpoint of stay on the skin and the like, and preferably 1 or less, more preferably 0.7 or less, even more preferably 0.5 or less, particularly preferably 0.3 or less, from the viewpoint of ease of production and the like. As a specific range, the mass ratio [(A)/(C)] is preferably 0.0005 or more and 1 or less, more preferably 0.0005 or more and 0.7 or less, even more preferably 0.001 or more and 0.5 or less, particularly preferably 0.001 or more and 0.3 or less.

The mass ratio of the component (B) to the component (C), [(B)/(C)], is preferably 0.0005 or more, more preferably 0.001 or more, particularly preferably 0.004 or more, from the viewpoint of rub resistance and the like, and preferably 1.5 or less, more preferably 0.5 or less, even more preferably 0.1 or less, particularly preferably 0.05 or less, from the viewpoint of a use impression (reliability in stay on the skin) and the like. Specifically, the mass ratio [(B)/(C)] is preferably 0.0005 or more and 1.5 or less, more preferably 0.001 or more and 0.5 or less, even more preferably 0.001 or more and 0.1 or less, particularly preferably 0.004 or more and 0.05 or less.

<Component (D)>

From the viewpoint of rub resistance, stay on the skin and the like, the sunscreen cosmetic of the present invention is preferably one comprising (D) a silicone-based film-forming agent in addition to the components (A) to (C).

In the present description, the (D) silicone-based film forming agent is an agent which has as a main chain a silicone segment having a chain structure or a branched structure and which has a film-forming ability.

Examples of the silicone-based film-forming agent include poly(N-acylalkyleneimine)-modified silicones (e.g. oxazoline-modified silicone), trimethylsiloxysilic acid and (trimethylsiloxysilic acid/dimethiconol) cross polymers. These may be used singly or in combinations of two or more thereof.

Of these, poly(N-acylalkyleneimine)-modified silicones are preferable, from the viewpoint of rub resistance, stay on the skin and the like.

(Poly(N-acylalkyleneimine)-modified silicone)

The poly(N-acylalkyleneimine)-modified silicone is preferably an organopolysiloxane in which a poly(N-acylalkyleneimine) segment consisting of repeating units of formula (9) is bonded via an alkylene group containing a hetero atom to at least two of silicon atoms of an organopolysiloxane segment forming a main chain, where the poly(N-acylalkyleneimine) segment has a number average molecular weight of 500 to 4,000, and the organopolysiloxane segment forming the main chain has a weight average molecular weight of 10,000 to 200,000 (hereinafter, the organopolysiloxane is also referred to as an organopolysiloxane (OX)).

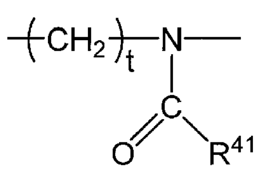
(9)

wherein $R^{41}$ represents a hydrogen atom, an alkyl group having 1 to 22 carbon atoms, an aralkyl group or an aryl group, and t represents 2 or 3.

The organopolysiloxane (OX) will now be described in detail.

At least two poly(N-acylalkyleneimine) segments are bonded via an alkylene group containing a hetero atom to arbitrary silicon atom forming the organopolysiloxane segment, preferably via the alkylene group to one or more silicon atoms other than those at both ends of the organopolysiloxane segment, more preferably via the alkylene group to two or more silicon atoms other than those at both ends of the organopolysiloxane segment.

The alkylene group containing a hetero atom functions as a linking group of the poly(N-acylalkyleneimine)

segment.

Examples of the alkylene group containing a hetero atom include alkylene groups having 2 to 20 carbon atoms and containing 1 to 3 nitrogen atoms, oxygen atoms or sulfur atoms. Of these, groups of any of formulae (A1) to (A10) are preferable, groups of any of formulae (A1) to (A4) are more preferable, and a group of formula (A1) or (A2) is particularly preferable.

In the formula, $An^-$ represents a counter ion of a quaternary ammonium salt. Examples thereof include halide ions (e.g. chloride ions and iodide ions), sulfate ions, phosphate ions, acetate ions, lactate ions, p-toluenesulfonate ions, perchlorate ions, and monoalkyl nitrate ions (e.g. methyl sulfate ions and ethyl sulfate ions).

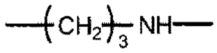
(A 1)

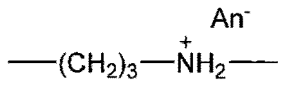
(A 2)

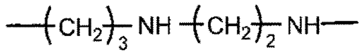
(A 3)

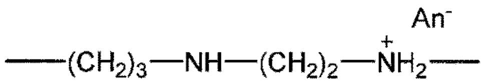
(A 4)

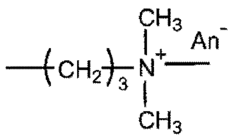
(A 5)

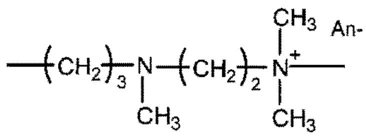
(A 6)

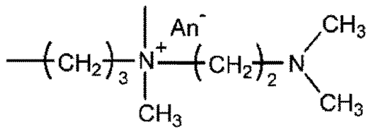
(A 7)

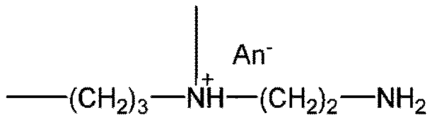
(A 8)

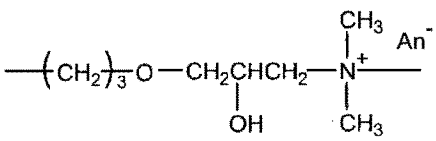
(A 9)

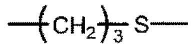
(A 1 0)

In the N-acylalkyleneimine unit forming the poly(N-acylalkyleneimine) segment, the number of carbon atoms in the alkyl group represented by $R^{41}$ in formula (9) is 1 to 22, preferably 1 to 14, more preferably 1 to 6, particularly preferably 1 to 3. The alkyl group may be linear or branched. Examples thereof include a methyl group, an ethyl group, a n-propyl group, an isopropyl group, a n-butyl group, a tert-butyl group, a pentyl group and a hexyl group.

The aralkyl group represented by $R^{41}$ is preferably an aralkyl group having 7 to 15 carbon atoms. Examples thereof include a benzyl group, a phenethyl group, a trityl group, a naphthylmethyl group and an anthracenylmethyl group.

The aryl group represented by $R^{41}$ is preferably an aryl group having 6 to 14 carbon atoms. Examples thereof include a phenyl group, a tolyl group, a xylyl group, a naphthyl group, a biphenyl group, an anthracenyl group and a phenanthryl group.

In particular, $R^{41}$ is preferably a hydrogen atom, or a linear or branched alkyl group having 1 to 3 carbon atoms, more preferably a linear or branched alkyl group having 1 to 3 carbon atoms.

t in formula (9) represents 2 or 3, preferably 2.

In the organopolysiloxane (OX), the weight average molecular weight of an organopolysiloxane segment

between adjacent poly(N-acylalkyleneimine) segments (hereinafter, also referred to as "MWg") is preferably 1 000 to 40 000, more preferably 1 500 to 30 000, particularly preferably 1 750 to 5 000, from the viewpoint of rub resistance and the like.

In the present description, the "organopolysiloxane segment between adjacent poly(N-acylalkyleneimine) segments" is a segment which is surrounded by dashed lines between two points, i.e. a bonding point of a poly(N-acylalkyleneimine) segment to an organopolysiloxane segment (bonding point A) and a bonding point of an adjacent poly(N-acylalkyleneimine) segment (bonding point B) and which consists of one $R^{42}SiO$ unit, one $R^{43}$ and (y + 1) $(R^{42})_2SiO$ units as shown in formula (10). The "poly (N-acylalkyleneimine) segment" is $-Z^{11}-R^{44}$ bonded to $R^{43}$.

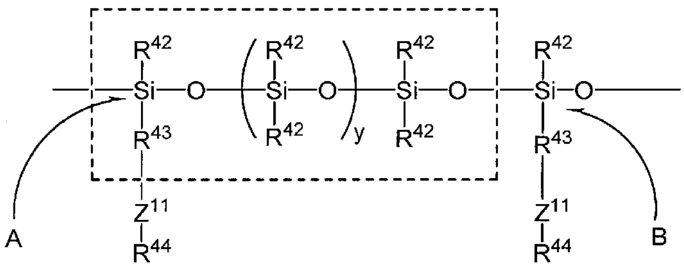

(10)

wherein $R^{42}$s each independently represent an alkyl group having 1 to 22 carbon atoms, or a phenyl group, $R^{43}$ represents an alkylene group containing a hetero atom, $-Z^{11}-R^{44}$ represents a poly (N-acylalkyleneimine) segment, $R^{44}$ represents a residue of a polymerization initiator, or a hydrogen atom, and y represents a positive number; and a plurality of $R^{42}$s, $R^{43}$s and $R^{44}$s may be the same or different.

MWg is a molecular weight of a segment surrounded by dashed lines in formula (10), and can be understood as a mass of the organopolysiloxane segment per mole of the poly (N-acylalkyleneimine) segment (g/mol). When functional groups of the modified organopolysiloxane as a raw material compound are replaced at 100% by poly(N-acylalkyleneimine) at 100%, MWg coincides with a functional group equivalent weight (g/mol) of the modified organopolysiloxane.

The molecular weight of the poly(N-acylalkyleneimine) segment can be calculated from the molecular weight of a N-acylalkyleneimine unit and the degree of polymerization, or measured by a gel permeation chromatography (GPC) method, and is herein a number average molecular weight in terms of polystyrene which is measured by GPC performed under measurement conditions described below (hereinafter, also referred by MNox). From the viewpoint of rub resistance and the like, MNox is preferably from 800 to 3 500, more preferably from 1 000 to 3 000.

MWg can be determined from formula (I) using a content ratio (mass%) of the organopolysiloxane segment forming the main chain (hereinafter, also referred to as Csi).

$$MWg = Csi \times MNox / (100 - Csi) \quad (I)$$

From the viewpoint of rub resistance and the like, the weight average molecular weight of the organopolysiloxane segment forming the main chain (hereinafter, also referred to as MWsi) is preferably from 15 000 to 160 000, more preferably from 20 000 to 150 000, particularly preferably from 25 000 to 100 000.

Since the organopolysiloxane segment forming the main chain has a backbone identical to that of the modified organopolysiloxane as a raw material compound, MWsi is substantially coincident with the weight average molecular weight of the modified organopolysiloxane as a raw material compound. The weight average molecular weight of the modified organopolysiloxane as a raw material compound is a weight average molecular weight in terms of polystyrene which is measured by GPC performed under the following measurement conditions.

(Conditions for measurement of weight average molecular weight of modified organopolysiloxane)

Column: Super HZ4000+Super HZ2000 (manufactured by TOSOH CORPORATION)
Eluent: 1 mM triethylamine/THF
Flow rate: 0.35 mL/min
Column temperature: 40°C
Detector: UV
Sample: 50 μL

From the viewpoint of rub resistance and the like, the weight average molecular weight of organopolysiloxane

(OX) (hereinafter, also referred to as MWt) is preferably from 12 000 to 200 000, more preferably from 25 000 to 160 000, particularly preferably from 35 000 to 150 000. MWt is a value in terms of polystyrene which is measured by GPC performed under measurement conditions described below.

(Conditions for measurement of MNox and MWt)

Column: two K-804Ls (manufactured by TOSOH CORPORATION) connected to each other in series
Eluent: 1 mM dimethyldodecylamine/chloroform
Flow rate: 1.0 mL/min
Column temperature: 40°C
Detector: RI
Sample: 50 μL

The poly(N-acylalkyleneimine) segment is a poly(N-propionylethyleneimine) segment, $^1$H-NMR measurement for calculation of the mass ratio (a/b) can be performed under, for example, the following conditions.
($^1$H-NMR measurement conditions)
0.5 g of a polymer sample dissolved in 2 g of a measurement solvent (deuterated chloroform) is measured by $^1$H-NMR (400 MHz, manufactured by Varian). The ratio of silicone to poly(N-propionylethyleneimine) is calculated from each integrated value.

PULSE SEQUENCE

- Relax. Delay: 30 seconds
- Pulse: 45 degrees
- Number of integrations: 8

Observed peak: methyl group of polydimethylsiloxane near 0 ppm and methylene moiety of ethyleneimine near 3.4 ppm

As the organopolysiloxane (OX), one obtained by performing synthesis in accordance with a method disclosed in JP-A-2008-143820, JP-A-2009-24114, JP-A-2015-67603 or the like may be used. For example, the organopolysiloxane (OX) can be produced by reacting an organopolysiloxane modified with a functional group, which directs the "alkylene group containing a hetero atom", with a terminally reactive poly(N-acylalkyleneimine) obtained by ring-opening polymerization of cyclic imino ether corresponding to a repeating unit of formula (9).
The content of the silicone-based film-forming agent is preferably 0.0001 mass% or more, more preferably 0.0005 mass% or more, even more preferably 0.001 mass% or more, particularly preferably 0.005 mass% or more, in the sunscreen cosmetic of the present invention, from the viewpoint of rub resistance, stay on the skin and the like, and preferably 5 mass% or less, more preferably 1 mass% or less, even more preferably 0.5 mass% or less, particularly preferably 0.2 mass% or less, in the sunscreen cosmetic of the present invention, from the viewpoint of rub resistance, stay on the skin, ease of production and the like. Specifically, the content of the silicone-based film-forming agent is preferably 0.0001 mass% or more and 5 mass% or less, more preferably 0.0005 mass% or more and 1 mass% or less, even more preferably 0.001 mass% or more and 0.5 mass% or less, particularly preferably 0.005 mass% or more and 0.2 mass% or less, in the sunscreen cosmetic of the present invention.
The mass ratio of the component (D) to the component (C), [(D)/(C)], is preferably 0.00001 or more, more preferably 0.0001 or more, particularly preferably 0.001 or more, from the viewpoint of rub resistance, stay on the skin and the like, and preferably 1 or less, more preferably 0.1 or less, particularly preferably 0.05 or less, from the viewpoint of ease of production, a use impression (high spreadability) and the like. Specifically, the mass ratio [(D)/(C)] is preferably 0.00001 or more and 1 or less, more preferably 0.0001 or more and 0.1 or less, particularly preferably 0.001 or more and 0.05 or less.
The sunscreen cosmetic of the present invention is an oil-in-water emulsion cosmetic comprising water and may comprise liquid oil agents other than organic UV absorbers which are liquid at 25°C at 1 atm (hereinafter, also referred to as "other liquid oil agents"); surfactants such as anionic surfactants, cationic surfactants, nonionic surfactants and ampholytic surfactants; powder other than hydrophobized UV scattering agents; waxes other than those described above; water-soluble polymers such as xanthan gum and (sodium acrylate/sodium acryloyldimethyltaurate) copolymers; monohydric

alcohols such as ethanol and propanol; polyhydric alcohols such as ethylene glycol, propylene glycol, 1,3-butylene glycol, glycerin, diglycerin and polyglycerin; chelating agents; preservatives; perfumes; pH adjusters; humectants; refrigerants; conditioning agents; and the like, in addition to the components described above. These may be used singly or in combinations of two or more thereof.

The content of water is preferably 5 mass% or more and 90 mass% or less, more preferably 7 mass% or more and 80 mass% or less, even more preferably 10 mass% or more and 70 mass% or less, particularly preferably 20 mass% or more and 70 mass% or less, in the sunscreen cosmetic of the present invention.

Other liquid oil agents are liquid oil agents other than organic UV absorbers among oil agents which are liquid at 25°C at 1 atm. The other liquid oil agents may be volatile oil agents or nonvolatile oil agents. These may be used singly or in combinations of two or more thereof. The volatile oil agent is an oil agent which is volatile at 25°C, and the nonvolatile oil agent is an oil agent which is nonvolatile at 25°C.

Examples of the volatile oil agent include light isoparaffin, decamethylcyclopentasiloxane, octamethylcyclotetrasiloxane, dodecamethylcyclohexasiloxane, methyl trimethicone, decamethyltetrasiloxane, ethyltrisiloxane and volatile methylpolysiloxane.

Examples of the commercialized product of the light isoparaffin include Isopar H (manufactured by Esso Chemical Company), Isododecane (manufactured by Bayer AG), Isohexadecane (manufactured by Uniqema Corporation), and IP Solvent 1620 MU, IP Solvent 2028 MU and IP Solvent 2835 (each manufactured by Idemitsu Kosan Co., Ltd.). Examples of the commercialized product of the decamethylcyclopentasiloxane include TFS405 (manufactured by Momentive Performance Materials Japan Ltd.), SH 245, DC 345 (manufactured by Dow Corning Toray Co., Ltd.) and KF-995 (manufactured by Shin-Etsu Chemical Co., Ltd.). Examples of the commercialized product of the methyl trimethicone include Silicone TMF-1.5 (manufactured by Shin-Etsu Chemical Co., Ltd.). Examples of the commercialized product of the decamethyltetrasiloxane include KF-96L-1.5 CS (manufactured by Shin-Etsu Chemical Co., Ltd.). Examples of the commercialized product of the ethyltrisiloxane include SILSOFTETS (manufactured by Momentive Performance Materials Japan Ltd.). Examples of the commercialized product of the volatile methylpolysiloxane include KF-96L-2CS (manufactured by Shin-Etsu Chemical Co., Ltd.).

Examples of the nonvolatile oil agent include nonvolatile hydrocarbon oils such as light liquid paraffin, liquid paraffin, heavy liquid isoparaffin and squalane; nonvolatile silicone oils such as nonvolatile dimethylpolysiloxane and nonvolatile methylphenylpolysiloxane; nonvolatile fatty acid ester oils such as cetyl 2-ethylhexanoate, isononyl isononanoate, isotridecyl isononanoate, isopropyl myristate, isopropyl palmitate, 2-ethylhexyl palmitate, 2-ethylhexyl stearate and stearyl stearate; and alkyl benzoates (C12-15). As the nonvolatile fatty acid ester oil, fatty acid triglycerides such as glyceryl tri(caprylate/caprate) and glyceryl tri(2-ethylhexanoate); esters of a fatty acid and neopentyl glycol such as neopentyl glycol dicaprate and neopentyl glycol diethylhexanoate; polyhydric alcohol fatty acid ester oils; and the like may be used.

Examples of the commercialized product of the liquid paraffin include Parleam 4 (manufactured by NOF CORPORATION). Examples of the commercialized product of the nonvolatile dimethylpolysiloxane include KF-96A-10 CS (manufactured by Shin-Etsu Chemical Co., Ltd.). Examples of the commercialized product of the isopropyl palmitate include Exeparl IPP (manufactured by Kao Corporation). Examples of the commercialized product of the alkyl benzoate (C12-15) include FINSOLVTN (Innospec Active Chemicals LLC). Examples of the commercialized product of the neopentyl glycol dicaprate include Estemol N-01 (manufactured by Nisshin OilliO Group, Ltd.).

The content of other liquid oil agents is preferably 0.5 mass% or more and 80 mass% or less, more preferably 1 mass% or more and 70 mass% or less, even more preferably 5 mass% or more and 60 mass% or less, particularly preferably 5 mass% or more and 50 mass% or less, in the sunscreen cosmetic of the present invention.

The sunscreen cosmetic of the present invention is an oil-in-water type emulsion and has excellent rub resistance, and exhibits a UV protection effect when the coating film is rubbed.

The sunscreen cosmetic of the present invention can be applied to the skin (preferably a skin other than the scalp, more preferably a face, a body, limbs and the like), and used as a sunscreen. Although the method for use of the sunscreen cosmetic is not particularly limited, it is preferable to apply the sunscreen cosmetic by hand or a tool for application.

The sunscreen cosmetic of the present invention can be produced in accordance with a conventional method.

The sunscreen cosmetic of the present invention reliably stay on the skin, has excellent rub resistance, and exhibits an excellent UV protection effect when the coating film is rubbed.

Regarding the embodiments described above, the present invention further discloses the following sunscreen cosmetics and so on.

<1> A sunscreen cosmetic comprising components (A), (B) and (C) and which is an oil-in-water emulsion cosmetic:

(A) a wax having a melting point of 55°C or higher in an amount of from 0.05 to 1.5 mass%, wherein the wax is selected the group consisting of a hydrocarbon-based wax and a silicone-based wax, and wherein the silicone-based wax is selected from the group consisting of alkyl methicone wax, alkyl dimethicone wax and silsesquioxane resin wax;
(B) a (meth)acryl-based silicone resin selected from the group consisting of a (meth)acryl-based polymer having a dendrimer-type siloxane structure at a side chain and a (meth)acryl-silicone-based graft copolymer; and
(C) a UV filter in an amount of from 2.5 to 45 mass%, wherein the UV filter is selected from the group consisting of a hydrophobized UV scattering agent and a UV absorber, wherein the hydrophobized UV scattering agent is a hydrophobized particulate metal oxide and the UV absorber is an organic UV absorber selected from the group consisting of a benzoic acid-based UV absorber, an anthranilic acid-based UV absorber, a salicylic acid-based UV absorber, a cinnamic acid-based UV absorber, a benzophenone-based UV absorber, and a triazine-based UV absorber.

<2> The sunscreen cosmetic according to <1>, wherein the melting point of the (A) wax is preferably 57.5°C or higher, more preferably 60°C or higher, and preferably 140°C or lower, more preferably 125°C or lower, particularly preferably 110°C or lower.
<3> The sunscreen cosmetic according to <1> or <2>, wherein the (A) wax is preferably a combination of a hydrocarbon-based wax and a silicone-based wax, or a silicone-based wax, particularly preferably a combination of a hydrocarbon-based wax and a silicone-based wax.
<4> The sunscreen cosmetic according to any one of <1> to <3>, wherein the hydrocarbon-based wax is preferably one or more selected from the group consisting of a mineral-based hydrocarbon wax, a petroleum-based hydrocarbon wax and a synthetic hydrocarbon wax, more preferably one or more selected from the group consisting of ozokerite, ceresin wax, paraffin wax, microcrystalline wax, Fisher Tropsch wax, polyethylene wax, and $\alpha$-olefins having 28 or more carbon atoms, even more preferably one or more selected from the group consisting of ceresin wax, paraffin wax, microcrystalline wax and a synthetic hydrocarbon wax, particularly preferably one or more selected from the group consisting of ceresin wax and a synthetic hydrocarbon wax.
<5> The sunscreen cosmetic according to any one of <1> to <4>, wherein the silicone-based wax is preferably an alkyl methicone wax.
<6> The sunscreen cosmetic according to any one of <1> to <5>, wherein the content of the component (A) is preferably 0.075 mass% or more, more preferably 0.1 mass% or more, in the sunscreen cosmetic of the present invention, and preferably 1.25 mass% or less, more preferably 1 mass% or less, in the sunscreen cosmetic of the present invention.
<7> The sunscreen cosmetic according to any one of <1> to <5>, wherein the content of the component (A) is preferably from 0.075 mass% to 1.25 mass%, more preferably from 0.1 mass% to 1 mass%, in the sunscreen cosmetic of the present invention.
<8> The sunscreen cosmetic according to any one of <1> to <7>, wherein the component (B) is preferably a (meth) acryl-based polymer having a dendrimer-type siloxane structure at a side chain, or is preferably a (meth)acryl-silicone-based graft copolymer, more preferably a (meth)acryl-based polymer having a dendrimer-type siloxane structure at a side chain.
<9> The sunscreen cosmetic according to <8>, wherein the (meth)acryl-based polymer having a dendrimer-type siloxane structure at a side chain is preferably a (meth)acryl-based polymer having a structural unit of formula (1), more preferably one in which the dendrimer-type siloxane structure denoted by D in formula (1) is a structure of formula (2), particularly preferably an (acrylates/polytrimethylsiloxy methacrylate) copolymer:

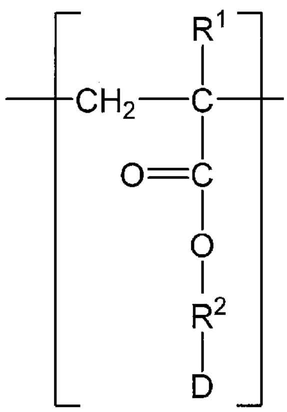

(1)

wherein

$R^1$ represents a hydrogen atom or a methyl group;

$R^2$ represents an alkanediyl group having 1 to 10 carbon atoms; and
D represents a dendrimer-type siloxane structure;

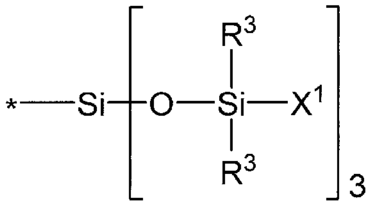

(2)

wherein

$R^3$ represents an alkyl group having 1 to 10 carbon atoms, or an aryl group;
$X^1$ represents a silylalkyl group of formula (3) in which i is 1; and
* represents a bond; and

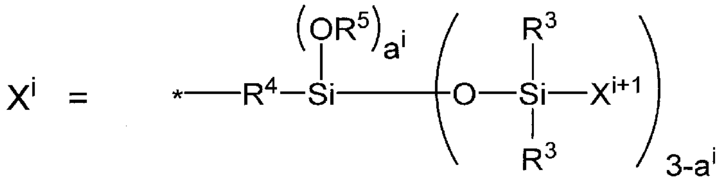

(3)

wherein

$R^3$ has the same meaning as described above;
$R^4$ represents an alkanediyl group having 2 to 10 carbon atoms;
$R^5$ represents an alkyl group having 1 to 10 carbon atoms;
$X^{i+1}$ represents a hydrogen atom, an alkyl group having 1 to 10 carbon atoms, an aryl group, or the same silylalkyl group as $X^i$;
$a^i$ represents an integer of 0 to 3; and
* represents a bond.

<10> The sunscreen cosmetic according to <8>, wherein the (meth)acryl-silicone-based graft copolymer is preferably one or more selected from the group consisting of an (acrylates/dimethicone) copolymer, an (acrylates/ethylhexyl acrylate/dimethicone methacrylate) copolymer, an (acrylates/stearyl acrylate/dimethicone methacrylate) copolymer and an (acrylates/behenyl acrylate/dimethicone methacrylate) copolymer.
<11> The sunscreen cosmetic according to any one of <1> to <10>, wherein a content of the component (B) is preferably 0.01 mass% or more, more preferably 0.05 mass% or more, even more preferably 0.1 mass% or more, particularly preferably 0.2 mass% or more, in the sunscreen cosmetic of the present invention, and preferably 10 mass% or less, more preferably 6 mass% or less, even more preferably 3 mass% or less, particularly preferably 1.5 mass% or less, in the sunscreen cosmetic of the present invention.
<12> The sunscreen cosmetic according to any one of <1> to <10>, wherein the content of the component (B) is preferably 0.01 mass% or more and 10 mass% or less, more preferably 0.05 mass% or more and 6 mass% or less, even more preferably 0.1 mass% or more and 3 mass% or less, even more preferably 0.1 mass% or more and 1.5 mass% or less, particularly preferably 0.2 mass% or more and 1.5 mass% or less, in the sunscreen cosmetic of the present invention.
<13> The sunscreen cosmetic according to any one of <1> to <12>, wherein the mass ratio of the component (A) to the component (B), [(A)/(B)], is preferably 0.01 or more, more preferably 0.05 or more, further more preferably 0.1 or more, even more preferably 0.4 or more, particularly preferably 0.55 or more, and preferably 10 or less, more preferably 5 or less, even more preferably 2.5 or less, particularly preferably 1.5 or less.
<14> The sunscreen cosmetic according to any one of <1> to <12>, wherein the mass ratio [(A)/(B)] is preferably 0.01 or more and 10 or less, more preferably 0.05 or more and 5 or less, further more preferably 0.1 or more and 2.5 or less, even more preferably 0.4 or more and 1.5 or less, particularly preferably 0.55 or more and 1.5 or less.
<15> The sunscreen cosmetic according to any one of <1> to <14>, wherein the component (C) is preferably a hydrophobized particulate metal oxide, wherein the particulate metal oxide is preferably one or more particulate metal oxides selected from the group consisting of particulate zinc oxide, particulate titanium oxide, particulate cesium

oxide, particulate iron oxide and particulate chromium oxide, more preferably one or more particulate metal oxides selected from the group consisting of particulate zinc oxide, particulate titanium oxide and particulate cesium oxide, even more preferably one or more particulate metal oxides selected from the group consisting of particulate zinc oxide and particulate titanium oxide, particularly preferably particulate zinc oxide.

<16> The sunscreen cosmetic according to any one of <15>, wherein the hydrophobization treatment is preferably one or more selected from the group consisting of fluorine compound treatment, silicone treatment, silicone resin treatment, pendant treatment, silane coupling agent treatment, titanium coupling agent treatment, oil agent treatment, N-acylated lysine treatment, polyacrylic acid treatment, metallic soap treatment, amino acid treatment, inorganic compound treatment, plasma treatment, mechanochemical treatment, silane compound treatment and silazane compound treatment, more preferably one or more selected from the group consisting of treatment with silicone or a silicone resin, treatment with a silane compound or a silazane compound, and metallic soap treatment.

<17> The sunscreen cosmetic according to any one of <1> to <14>, wherein the UV absorber is preferably an organic UV absorber selected from the group consisting of a benzoic acid-based UV absorber, an anthranilic acid-based UV absorber, a salicylic acid-based UV absorber, a cinnamic acid-based UV absorber, a benzophenone-based UV absorber, a and triazine-based UV absorber, even more preferably one or more selected from the group consisting of a cinnamic acid-based UV absorber and a triazine-based UV absorber.

<18> The sunscreen cosmetic according to any one of <1> to <17>, wherein the content of the component (C) is preferably 5 mass% or more, more preferably 7 mass% or less, particularly preferably 10 mass% or more, in the sunscreen cosmetic of the present invention, and preferably 40 mass% or less, more preferably 35 mass% or less, particularly preferably 30 mass% or less, in the sunscreen cosmetic of the present invention.

<19> The sunscreen cosmetic according to any one of <1> to <17>, wherein the content of the component (C) is preferably 5 mass% or more and 40 mass% or less, more preferably 7 mass% or more and 35 mass% or less, particularly preferably 10 mass% or more and 30 mass% or less, in the sunscreen cosmetic of the present invention.

<20> The sunscreen cosmetic according to any one of <1> to <19>, wherein the mass ratio of the component (A) to the component (C), [(A)/(C)], is preferably 0.0005 or more, more preferably 0.001 or more, and preferably 1 or less, more preferably 0.7 or less, even more preferably 0.5 or less, particularly preferably 0.3 or less.

<21> The sunscreen cosmetic according to any one of <1> to <20>, wherein the mass ratio of the component (B) to the component (C), [(B)/(C)], is preferably 0.0005 or more, more preferably 0.001 or more, particularly preferably 0.004 or more, and preferably 1.5 or less, more preferably 0.5 or less, even more preferably 0.1 or less, particularly preferably 0.05 or less.

<22> The sunscreen cosmetic according to any one of <1> to <21>, further comprising (D) a silicone-based film-forming agent, wherein the component (D) is a poly(N-acylalkyleneimine)-modified silicone.

<23> The sunscreen cosmetic according to <22>, wherein the poly(N-acylalkyleneimine)-modified silicone is preferably an organopolysiloxane in which a poly(N-acylalkyleneimine) segment consisting of repeating units of formula (9) is bonded via an alkylene group containing a hetero atom to at least two of silicon atoms of an organopolysiloxane segment forming a main chain, where the poly(N-acylalkyleneimine) segment has a number average molecular weight of 500 to 4 000, and the organopolysiloxane segment forming the main chain has a weight average molecular weight of 10 000 to 200 000:

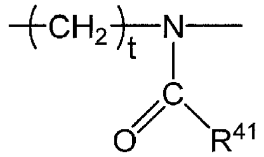
(9)

wherein $R^{41}$ represents a hydrogen atom, an alkyl group having 1 to 22 carbon atoms, an aralkyl group or an aryl group, and t represents 2 or 3.

<24> The sunscreen cosmetic according to <22> or <23>, wherein the content of the component (D) is preferably 0.0001 mass% or more, more preferably 0.0005 mass% or more, even more preferably 0.001 mass% or more, particularly preferably 0.005 mass% or more, in the sunscreen cosmetic of the present invention, and preferably 5 mass% or less, more preferably 1 mass% or less, even more preferably 0.5 mass% or less, particularly preferably 0.2 mass% or less, in the sunscreen cosmetic of the present invention.

<25> The sunscreen cosmetic according to <22> or <23>, wherein the content of the component (D) is preferably 0.0001 mass% or more and 5 mass% or less, more preferably 0.0005 mass% or more and 1 mass% or less, even more preferably 0.001 mass% or more and 0.5 mass% or less, particularly preferably 0.005 mass% or more and 0.2 mass% or less, in the sunscreen cosmetic of the present invention.

<26> The sunscreen cosmetic according to any one of <22> to <25>, wherein the mass ratio of the component (D) to the component (C), [(D)/(C)], is preferably 0.00001 or more, more preferably 0.0001 or more, particularly 0.001 or

more, and preferably 1 or less, more preferably 0.1 or less, particularly preferably 0.05 or less.

<27> The sunscreen cosmetic according to any one of <1> to <26>, further comprising one or more selected from the group consisting of a liquid oil agent other than organic UV absorbers which are liquid at 25°C at 1 atm; a surfactant; powder other than hydrophobized UV scattering agents; a wax other than the waxes described above; a water-soluble polymer; a monohydric alcohol; a polyhydric alcohols; a chelating agent; a preservative; a perfume; a pH adjuster; a humectant; a refrigerant; and a conditioning agent.

<28> The sunscreen cosmetic according to <1> to <27>, wherein the content of water is preferably 5 mass% or more and 90 mass% or less, more preferably 7 mass% or more and 80 mass% or less, even more preferably 10 mass% or more and 70 mass% or less, particularly preferably 20 mass% or more and 70 mass% or less, in the sunscreen cosmetic of the present invention.

<29> The sunscreen cosmetic according to <27> or <28>, wherein the content of the liquid oil agent other than the organic UV absorbers which are liquid at 25°C at 1 atm is preferably 0.5 mass% or more and 80 mass% or less, more preferably 1 mass% or more and 70 mass% or less, even more preferably 5 mass% or more and 60 mass% or less, particularly preferably 5 mass% or more and 50 mass% or less, in the sunscreen cosmetic of the present invention.

## Examples

Hereinafter, the present invention will be described in detail by way of Examples. In Examples, various measurements and evaluations were performed by the following methods.

• Evaluation method

(1) Use impression (reliable stay on skin)

Ten professional panelists performed sensory evaluation regarding stay on the skin. The evaluation criteria are shown below. The "impression of reliable stay on the skin" refers to an impression of no slippery feeling during application and no slimy or sticky feeling on the skin with stabilized condition a certain period of time after the application.

(Evaluation criteria for use impression)

AA: Nine or more panelists perceived no slippery feeling during application and no slimy and sticky feeling after the application.

A: Eight panelists perceived no slippery feeling during application and no slimy and sticky feeling after the application.

B+: Five to seven panelists perceived no slippery feeling during application and no slimy and sticky feeling after the application.

B-: Three or four panelists perceived no slippery feeling during application and no slimy and sticky feeling after the application.

C: Two or less panelists perceived no slippery feeling during application and no slimy and sticky feeling after the application.

(2) UV protection effect and rub resistance

Each cosmetic was uniformly applied to a PMMA plate at 1.3 mg/cm$^2$, and then naturally dried for 15 minutes, and SPF was measured using a SPF analyzer (Labsphere UV-2000S from Optmetrics Corporation) (SPF before rubbing).

Thereafter, a cosmetic application part of the PMMA plate was rubbed by applying a load of 100 g to a terminal provided with a 2 $\times$ 2 cm$^2$ cotton cloth using a rub tester (Tribomaster from Trinity-lab. Inc.). SPF after rubbing was measured with the SPF analyzer (SPF after rubbing), and the SPF residual ratio was calculated from the formula below to evaluate rub resistance. The higher the SPF residual ratio is, the higher the rub resistance is.

(SPF residual ratio (%)) = {(SPF after rubbing)/(SPF before rubbing)} $\times$ 100

(Examples 1 to 19 and Comparative Examples 1 to 4: Oil-in-water emulsion cosmetic; Example 5 is a Reference Example)

The oil-in-water cosmetics of Examples 1 to 19 and Comparative Examples 1 to 4 were produced by a conventional method in accordance with formulations shown in Tables 1 to 4, and were evaluated for the use impression

(reliability in stay on the skin), the UV protection effect and the rub resistance. Tables 1 to 4 show the results.

[Table 1]

| Component (mass%) | | Example 1 | Example 2 | Example 3 | Example 4 | Ref. Ex. 5 |
|---|---|---|---|---|---|---|
| A | Mixture of C30-45 alkyl methicone and C30-45 olefin (melting point: 73 to 77°C) *1 | 1.000 | 1.000 | 1.000 | 1.000 | 1.000 |
| A | C30-45 alkyl dimethylsilyl polypropylsilsesquioxane (melting point: 63 to 71°C) *2 | - | - | - | - | - |
| A | Paraffin wax 155 (melting point: 69°C) *3 | - | - | - | - | - |
| A | Paraffin wax HNP-9 (melting point 75°C) *4 | - | - | - | - | - |
| A | LIPWAX A-4 (melting point: 92 to 104°C) *5 | - | - | - | - | - |
| A | Multiwax W-445 S (melting point 76.7 to 82.4°C) *6 | - | - | - | - | - |
| A | Ceresin wax SP-1020P (melting point: 73.3 to 76.1°C) *7 | - | - | - | - | - |
| A' | Stearyl dimethicone (melting point: 28 to 35°C) *8 | - | - | - | - | - |
| B | (Acrylates/polytrimethylsiloxy methacrylate) copolymer *9 | 0.800 | 0.800 | - | 0.800 | 0.800 |
| B | (Acrylates/dimethicone) copolymer *10 | - | - | 0.800 | - | - |
| C | Ethylhexyl methoxycinnamate | 8.000 | 1.670 | 8.000 | 8.000 | 8.000 |
| C | Ethylhexyl triazone | 2.000 | 0.420 | 2.000 | 2.000 | 2.000 |
| C | Bis-ethylhexyloxyphenol methoxyphenyl triazine | 2.000 | 0.420 | 2.000 | 2.000 | 2.000 |
| C | Triethoxycaprylylsilane-treated particulate zinc oxide | 12.000 | 2.500 | 12.000 | 12.000 | 12.000 |
| D | Poly(N-propionylethyleneimine)-modified silicone *11 | 0.100 | 0.100 | 0.100 | - | - |
| D | Polypropylsilsesquioxane *12 | - | - | - | - | 0.100 |
| | Isononyl isononanoate | 4.500 | 4.500 | 4.500 | 4.500 | 4.500 |
| | Dimethicone (2cs) | 5.100 | 5.100 | 5.100 | 5.100 | 5.100 |
| | Cyclopentasiloxane | - | - | 1.890 | - | 0.096 |
| | Sodium acrylate-sodium acryloyldimethyltaurate copolymer/isohexadecane/Polysorbate 80 | 2.000 | 2.000 | 2.000 | 2.000 | 2.000 |
| | Xanthan qum | 0.100 | 0.100 | 0.100 | 0.100 | 0.100 |
| | 1,3-Butylene glycol | 2.000 | 2.000 | 2.000 | 2.000 | 2.000 |
| | Glycerin | 2.000 | 2.000 | 2.000 | 2.000 | 2.000 |
| | EDTA-2Na | 0.020 | 0.020 | 0.020 | 0.020 | 0.020 |
| | Ethanol | 10.000 | 10.000 | 10.000 | 10.000 | 10.000 |

(continued)

| Component (mass%) | | Example | | | | |
|---|---|---|---|---|---|---|
| | | 1 | 2 | 3 | 4 | Ref. Ex. 5 |
| | Water | Balance | Balance | Balance | Balance | Balance |
| Total | | 100 | 100 | 100 | 100 | 100 |
| Mass ratio [(A)/(B)] | | 1.25 | 1.25 | 1.25 | 1.25 | 1.25 |
| Evaluation Result | SPF before rubbing | 68.46 | 25.19 | 69.06 | 69.19 | 71.33 |
| | SPF after rubbing | 58.66 | 18.06 | 50.53 | 49.72 | 52.59 |
| | SPF residual ratio (%) | 85.69 | 71.69 | 73.17 | 71.86 | 73.73 |
| | Use impression (reliability in stay on skin) | AA | A | A | B+ | B+ |

[Table 2]

| Component (mass%) | | Example | | | | | |
|---|---|---|---|---|---|---|---|
| | | 6 | 7 | 8 | 9 | 10 | 11 |
| A | Mixture of C30-45 alkyl methicone and C30-45 olefin (melting point: 73 to 77°C) *1 | - | - | - | - | - | - |
| A | C30-45 alkyl dimethylsilyl polypropylsilsesquioxane (melting point: 63 to 71°C) *2 | 1.000 | - | - | - | - | - |
| A | Paraffin wax 155 (melting point: 69°C) *3 | - | 1.000 | - | - | - | - |
| A | Paraffin wax HNP-9 (melting point 75°C) *4 | - | - | 1.000 | - | - | - |
| A | LIPWAX A-4 (melting point: 92 to 104°C) *5 | - | - | - | 1.000 | - | - |
| A | Multiwax W-445 S (melting point 76.7 to 82.4°C) *6 | - | - | - | - | 1.000 | - |
| A | Ceresin wax SP-1020P (melting point: 73.3 to 76.1°C) *7 | - | - | - | - | - | 1.000 |
| A' | Stearyl dimethicone (melting point: 28 to 35°C) *8 | - | - | - | - | - | - |
| B | (Acrylates/polytrimethylsiloxy methacrylate) copolymer *9 | 0.800 | 0.800 | 0.800 | 0.800 | 0.800 | 0.800 |
| B | (Acrylates/dimethicone) copolymer *10 | - | - | - | - | - | - |
| C | Ethylhexyl methoxycinnamate | 8.000 | 8.000 | 8.000 | 8.000 | 8.000 | 8.000 |
| C | Ethylhexyl triazone | 2.000 | 2.000 | 2.000 | 2.000 | 2.000 | 2.000 |
| C | Bis-ethylhexyloxyphenol methoxyphenyl triazine | 2.000 | 2.000 | 2.000 | 2.000 | 2.000 | 2.000 |
| C | Triethoxycaprylylsilane-treated particulate zinc oxide | 12.000 | 12.000 | 12.000 | 12.000 | 12.000 | 12.000 |
| D | Poly(N-propionylethyleneimine)-modified silicone *11 | 0.100 | 0.100 | 0.100 | 0.100 | 0.100 | 0.100 |
| D | Polypropylsilsesquioxane *12 | - | - | - | - | - | - |
| | Isononyl isononanoate | 4.500 | 4.500 | 4.500 | 4.500 | 4.500 | 4.500 |
| | Dimethicone (2cs) | 5.100 | 5.100 | 5.100 | 5.100 | 5.100 | 5.100 |
| | Cyclopentasiloxane | - | - | - | - | - | - |
| | Sodium acrylate-sodium acryloyldimethyltaurate copolymer/isohexadecane/Polysorbate 80 | 2.000 | 2.000 | 2.000 | 2.000 | 2.000 | 2.000 |
| | Xanthan qum | 0.100 | 0.100 | 0.100 | 0.100 | 0.100 | 0.100 |
| | 1,3-Butylene glycol | 2.000 | 2.000 | 2.000 | 2.000 | 2.000 | 2.000 |
| | Glycerin | 2.000 | 2.000 | 2.000 | 2.000 | 2.000 | 2.000 |
| | EDTA-2Na | 0.020 | 0.020 | 0.020 | 0.020 | 0.020 | 0.020 |
| | Ethanol | 10.000 | 10.000 | 10.000 | 10.000 | 10.000 | 10.000 |

(continued)

| Component (mass%) | | Example | | | | | |
|---|---|---|---|---|---|---|---|
| | | 6 | 7 | 8 | 9 | 10 | 11 |
| | Water | Balance | Balance | Balance | Balance | Balance | Balance |
| Total | | 100 | 100 | 100 | 100 | 100 | 100 |
| Mass ratio [(A)/(B)] | | 1.25 | 1.25 | 1.25 | 1.25 | 1.25 | 1.25 |
| Evaluation Result | SPF before rubbing | 71.88 | 72.31 | 72.99 | 69.01 | 71.01 | 68.78 |
| | SPF after rubbing | 50.88 | 54.60 | 52.42 | 51.20 | 50.94 | 54.18 |
| | SPF residual ratio (%) | 70.78 | 75.51 | 71.82 | 74.19 | 71.74 | 78.77 |
| | Use impression (reliability in stay on skin) | AA | AA | AA | A | A | AA |

[Table 3]

| Component (mass%) | | Example | | | | | | | |
|---|---|---|---|---|---|---|---|---|---|
| | | 12 | 13 | 14 | 15 | 16 | 17 | 18 | 19 |
| A | Mixture of C30-45 alkyl methicone and C30-45 olefin (melting point: 73 to 77°C) *1 | 0.100 | 1.500 | 1.000 | 1.000 | 1.000 | 1.000 | 1.000 | 1.000 |
| A | C30-45 alkyl dimethylsilyl polypropylsilsesquioxane (melting point: 63 to 71°C) *2 | - | - | - | - | - | - | - | - |
| A | Paraffin wax 155 (melting point: 69°C) *3 | - | - | - | - | - | - | - | - |
| A | Paraffin wax HNP-9 (melting point 75°C) *4 | - | - | - | - | - | - | - | - |
| A | LIPWAX A-4 (melting point: 92 to 104°C) *5 | - | - | - | - | - | - | - | - |
| A | Multiwax W-445 S (melting point 76.7 to 82.4°C) *6 | - | - | - | - | - | - | - | - |
| A | Ceresin wax SP-1020P (melting point: 73.3 to 76.1°C) *7 | - | - | - | - | - | - | - | - |
| A' | Stearyl dimethicone (melting point: 28 to 35°C) *8 | - | - | - | - | - | - | - | - |
| B | (Acrylates/polytrimethylsiloxy methacrylate) copolymer *9 | 0.800 | 0.800 | 0.120 | 2.000 | 4.000 | 5.000 | 0.800 | 0.800 |
| B | (Acrylates/dimethicone) copolymer *10 | - | - | - | - | - | - | - | - |
| C | Ethylhexyl methoxycinnamate | 8.000 | 8.000 | 8.000 | 8.000 | 8.000 | 8.000 | 8.000 | 8.000 |
| C | Ethylhexyl triazone | 2.000 | 2.000 | 2.000 | 2.000 | 2.000 | 2.000 | 2.000 | 2.000 |
| C | Bis-ethylhexyloxyphenol methoxyphenyl triazine | 2.000 | 2.000 | 2.000 | 2.000 | 2.000 | 2.000 | 2.000 | 2.000 |
| C | Triethoxycaprylylsilane-treated particulate zinc oxide | 12.000 | 12.000 | 12.000 | 12.000 | 12.000 | 12.000 | 12.000 | 12.000 |
| D | Poly(N-propionylethyleneimine)-modified silicone *11 | 0.100 | 0.100 | 0.100 | 0.100 | 0.100 | 0.100 | 0.001 | 0.300 |
| D | Polypropylsilsesquioxane *12 | - | - | - | - | - | - | - | - |
| | Isononyl isononanoate | 4.500 | 4.500 | 4.500 | 4.500 | 4.500 | 4.500 | 4.500 | 4.500 |
| | Dimethicone (2cs) | 5.100 | 5.100 | 5.100 | 5.100 | 6.900 | 8.400 | 5.100 | 5.100 |
| | Cyclopentasiloxane | - | - | - | - | - | - | - | - |
| | Sodium acrylate-sodium acryloyldimethyltaurate copolymer/isohexadecane/Polysorbate 80 | 2.000 | 2.000 | 2.000 | 2.000 | 2.000 | 2.000 | 2.000 | 2.000 |
| | Xanthan gum | 0.100 | 0.100 | 0.100 | 0.100 | 0.100 | 0.100 | 0.100 | 0.100 |
| | 1,3-Butylene glycol | 2.000 | 2.000 | 2.000 | 2.000 | 2.000 | 2.000 | 2.000 | 2.000 |

(continued)

| Component (mass%) | | Example | | | | | | | |
|---|---|---|---|---|---|---|---|---|---|
| | | 12 | 13 | 14 | 15 | 16 | 17 | 18 | 19 |
| | Glycerin | 2.000 | 2.000 | 2.000 | 2.000 | 2.000 | 2.000 | 2.000 | 2.000 |
| | EDTA-2Na | 0.020 | 0.020 | 0.020 | 0.020 | 0.020 | 0.020 | 0.020 | 0.020 |
| | Ethanol | 10.000 | 10.000 | 10.000 | 10.000 | 10.000 | 10.000 | 10.000 | 10.000 |
| | Water | Balance | Balance | Balance | Balance | Balance | Balance | Balance | Balance |
| Total | | 100 | 100 | 100 | 100 | 100 | 100 | 100 | 100 |
| Mass ratio [(A)/(B)] | | 0.13 | 1.88 | 8.33 | 0.50 | 0.25 | 0.20 | 1.25 | 1.25 |
| Evaluation Result | SPF before rubbing | 72.58 | 75.11 | 76.15 | 73.65 | 72.38 | 69.38 | 75.26 | 69.19 |
| | SPF after rubbing | 54.33 | 55.32 | 59.24 | 51.63 | 52.22 | 50.98 | 54.88 | 49.72 |
| | SPF residual ratio (%) | 74.86 | 74.86 | 77.79 | 70.10 | 72.15 | 73.48 | 72.92 | 71.86 |
| | Use impression (reliability in stay on skin) | AA | A | AA | A | B- | B- | A | A |

[Table 4]

| Component (mass%) | | Comparative Example | | | |
|---|---|---|---|---|---|
| | | 1 | 2 | 3 | 4 |
| A | Mixture of C30-45 alkyl methicone and C30-45 olefin (melting point: 73 to 77°C) *1 | - | - | - | 2.000 |
| A | C30-45 alkyl dimethylsilyl polypropylsilsesquioxane (melting point: 63 to 71°C) *2 | - | - | - | - |
| A | Paraffin wax 155 (melting point: 69°C) *3 | - | - | - | - |
| A | Paraffin wax HNP-9 (melting point 75°C) *4 | - | - | - | - |
| A | LIPWAX A-4 (melting point: 92 to 104°C) *5 | - | - | - | - |
| A | Multiwax W-445 S (melting point 76.7 to 82.4°C) *6 | - | - | - | - |
| A | Ceresin wax SP-1020P (melting point: 73.3 to 76.1°C) *7 | - | - | - | - |
| A' | Stearyl dimethicone (melting point: 28 to 35°C) *8 | - | - | 1.000 | - |
| B | (Acrylates/polytrimethylsiloxy methacrylate) copolymer *9 | 0.800 | - | 0.800 | 0.800 |
| B | (Acrylates/dimethicone) copolymer *10 | - | - | - | - |
| C | Ethylhexyl methoxycinnamate | 8.000 | 8.000 | 8.000 | 8.000 |
| C | Ethylhexyl triazone | 2.000 | 2.000 | 2.000 | 2.000 |
| C | Bis-ethylhexyloxyphenol methoxyphenyl triazine | 2.000 | 2.000 | 2.000 | 2.000 |
| C | Triethoxycaprylylsilane-treated particulate zinc oxide | 12.000 | 12.000 | 12.000 | 12.000 |
| D | Poly(N-propionylethyleneimine)-modified silicone *11 | 0.100 | 0.100 | 0.100 | 0.100 |
| D | Polypropylsilsesquioxane *12 | - | - | - | - |
| | Isononyl isononanoate | 4.500 | 4.500 | 4.500 | 4.500 |
| | Dimethicone (2cs) | 5.100 | 5.100 | 5.100 | 5.100 |
| | Cyclopentasiloxane | - | - | - | - |
| | Sodium acrylate-sodium acryloyldimethyltaurate copolymer/isohexadecane/Polysorbate 80 | 2.000 | 2.000 | 2.000 | 2.000 |
| | Xanthan qum | 0.100 | 0.100 | 0.100 | 0.100 |
| | 1,3-Butylene glycol | 2.000 | 2.000 | 2.000 | 2.000 |
| | Glycerin | 2.000 | 2.000 | 2.000 | 2.000 |
| | EDTA-2Na | 0.020 | 0.020 | 0.020 | 0.020 |
| | Ethanol | 10.000 | 10.000 | 10.000 | 10.000 |
| | Water | Balance | Balance | Balance | Balance |
| Total | | 100 | 100 | 100 | 100 |
| Mass ratio [(A)/(B)] | | 0 | - | 0 | 2.50 |
| Evaluation Result | SPF before rubbing | 61.75 | 68.18 | 66.03 | 82.82 |
| | SPF after rubbing | 38.71 | 37.83 | 36.04 | 59.22 |
| | SPF residual ratio (%) | 62.69 | 55.49 | 54.58 | 71.50 |
| | Use impression (reliability in stay on skin) | C | C | C | C |

The symbols in the tables denote the following.

*1: AMS-C30 Cosmetic Wax (melting point: 73 to 77°C, manufactured by Dow Corning Toray Co., Ltd.); mixture of C30-45 alkyl methicone in an amount of 54 mass% and C30-45 olefin at in an amount of 46 mass%.
*2: SW-8005 C30 Resin Wax (melting point: 63 to 71°C, manufactured by Dow Corning Toray Co., Ltd.)
*3: Paraffin Wax 155 (melting point: 69°C, manufactured by NIPPON SEIRO CO., LTD.)
*4: Paraffin Wax HNP-9 (melting point: 75°C, manufactured by NIPPON SEIRO CO., LTD.)
*5: LIPWAX A-4 (melting point: 92 to 104°C, manufactured by Japan Natural Products Co., Ltd.)
*6: Multiwax W-445 S (melting point: 76.7 to 82.4°C, manufactured by Sonneborn LLC.)
*7: Ceresin Wax SP-1020P (melting point: 73.3 to 76.1°C, manufactured by Strahl & Pitsch, Inc.)
*8: 2503 Cosmetic Wax (melting point: 28 to 35°C, manufactured by Dow Corning Toray Co., Ltd.)
*9: (Acrylates/polytrimethylsiloxy methacrylate) copolymer-FA 4003 DM (manufactured by Dow Corning Toray Co., Ltd.)
*10: (Acrylates/dimethicone) copolymer KP-545 (manufactured by Shin-Etsu Chemical Co., Ltd.)
*11: Compound prepared as described in JP-A-2015-168663, Synthesis Example 1
*12: Polypropylsilsesquioxane 670 Fluid (manufactured by Dow Corning Toray Co., Ltd.)

(Reference Example 20: Water-in-oil emulsion cosmetic)

The water-in-oil emulsion cosmetic of Example 20 was produced by a conventional method in accordance with the formulation shown below, and was evaluated for the use impression (reliability in stay on the skin), the UV protection effect and the rub resistance. Table 5 shows the results.

[Table 5]

| Component (mass%) | | Ref. Example 20 |
|---|---|---|
| A | Mixture of C30-45 alkyl methicone and C30-45 olefin *1 | 1.0 |
| B | (Acrylates/polytrimethylsiloxy methacrylate) copolymer *9 | 0.8 |
| C | Ethylhexyl methoxycinnamate | 10.0 |
| C | Ethylhexyl triazone | 0.5 |
| C | Bis-ethylhexyloxyphenol methoxyphenyl triazine | 2.0 |
| C | Dimethicodiethylbenzalmalonate | 1.0 |
| C | Hexyl diethylaminohydroxybenzoylbenzoate | 0.5 |
| C | Triethoxycaprylylsilane-treated particulate zinc oxide | 15.0 |
| C | Triethoxycaprylylsilane-treated particulate titanium oxide | 5.0 |
| D | Poly(N-propionylethyleneimine)-modified silicone *11 | 0.1 |
| | PEG-8 trifluoropropyl dimethicone copolymer (FPD-6131 manufactured by Shin-Etsu Chemical Co., Ltd.) | 2.0 |
| | Sorbitan isostearate | 1.0 |
| | Diisopropyl sebacate | 3.0 |
| | Isononyl isononanoate | 2.0 |
| | Methyl trimethicone (TMF-1.5 manufactured by Shin-Etsu Chemical Co., Ltd.) | 19.5 |
| | Dimethicone (2cs) | 10.5 |
| | Ethanol | 7.0 |
| | Glycerin | 1.0 |
| | Water | 18.1 |
| Total | | 100.0 |
| Mass ratio [(A)/(B)] | | 1.25 |

(continued)

| Component (mass%) | | Ref. Example 20 |
|---|---|---|
| Evaluation Result | SPF before rubbing | 97.64 |
| | SPF after rubbing | 74.86 |
| | SPF residual ratio (%) | 76.67 |
| | Use impression (reliability in stay on skin) | AA |

## Claims

1. A sunscreen cosmetic comprising the following components (A), (B) and (C) and which is an oil-in-water emulsion cosmetic:

   (A) a wax having a melting point of 55°C or higher in an amount of from 0.05 to 1.5 mass%, wherein the wax is selected the group consisting of a hydrocarbon-based wax and a silicone-based wax, and wherein the silicone-based wax is selected from the group consisting of alkyl methicone wax, alkyl dimethicone wax and silsesquioxane resin wax;
   (B) a (meth)acryl-based silicone resin selected from the group consisting of a (meth)acryl-based polymer having a dendrimer-type siloxane structure at a side chain and a (meth)acryl-silicone-based graft copolymer; and
   (C) a UV filter in an amount of from 2.5 to 45 mass%, wherein the UV filter is selected from the group consisting of a hydrophobized UV scattering agent and a UV absorber, wherein the hydrophobized UV scattering agent is a hydrophobized particulate metal oxide and the UV absorber is an organic UV absorber selected from the group consisting of a benzoic acid-based UV absorber, an anthranilic acid-based UV absorber, a salicylic acid-based UV absorber, a cinnamic acid-based UV absorber, a benzophenone-based UV absorber, and a triazine-based UV absorber.

2. The sunscreen cosmetic according to claim 1, wherein a content of the component (B) is from 0.01 mass% to 10 mass% in relation to the sunscreen cosmetic.

3. The sunscreen cosmetic according to claim 1 or 2, wherein a content of the component (B) is from 0.1 mass% to 3 mass% in relation to the sunscreen cosmetic.

4. The sunscreen cosmetic according to any one of claims 1 to 3, further comprising (D) a silicone-based film-forming agent that is a poly(N-acylalkyleneimine)-modified silicone.

## Patentansprüche

1. Sonnenschutzkosmetikum, umfassend die folgenden Komponenten (A), (B) und (C) und das ein Öl-in-Wasser-Emulsionskosmetikum ist:

   (A) ein Wachs mit einem Schmelzpunkt von 55°C oder höher in einer Menge von 0,05 bis 1,5 Massen-%, wobei das Wachs aus der Gruppe ausgewählt ist, die aus einem Kohlenwasserstoff-basierten Wachs und einem Silikonbasierten Wachs besteht, und wobei das Silikon-basierte Wachs aus der Gruppe ausgewählt ist, die aus Alkylmethiconwachs, Alkyldimethiconwachs und Silsesquioxanharzwachs besteht;
   (B) ein (Meth)acryl-basiertes Silikonharz, das aus der Gruppe ausgewählt ist, die aus einem (Meth)acryl-basierten Polymer mit einer Siloxanstruktur vom Dendrimer-Typ an einer Seitenkette und einem (Meth)acryl-Silikon-basierten Pfropfcopolymer besteht; und
   (C) einen UV-Filter in einer Menge von 2,5 bis 45 Massen-%, wobei der UV-Filter aus der Gruppe ausgewählt ist, die aus einem hydrophobierten UV-Streumittel und einem UV-Absorber besteht, wobei das hydrophobierte UV-Streumittel ein hydrophobiertes partikelförmiges Metalloxid ist und der UV-Absorber ein organischer UV-Absorber ist, der aus der Gruppe ausgewählt ist, die aus einem Benzoesäure-basierten UV-Absorber, einem Anthranilsäure-basierten UV-Absorber, einem Salicylsäure-basierten UV-Absorber, einem Zimtsäurebasierten UV-Absorber, einem Benzophenon-basierten UV-Absorber und einem Triazin-basierten UV-Absorber besteht.

2. Sonnenschutzkosmetikum gemäß Anspruch 1, wobei ein Gehalt der Komponente (B) 0,01 Massen-% bis 10 Massen-%, bezogen auf das Sonnenschutzkosmetikum, beträgt.

3. Sonnenschutzkosmetikum gemäß Anspruch 1 oder 2, wobei ein Gehalt der Komponente (B) 0,1 Massen-% bis 3 Massen-%, bezogen auf das Sonnenschutzkosmetikum, beträgt.

4. Sonnenschutzkosmetikum gemäß einem der Ansprüche 1 bis 3, ferner umfassend (D) ein Silikon-basiertes filmbildendes Mittel, das ein Poly(N-acylalkylenimin)-modifiziertes Silikon ist.

## Revendications

1. Produit cosmétique d'écran solaire comprenant les composants suivants (A), (B) et (C) et qui est un cosmétique à émulsion huile-dans-eau :

   (A) une cire ayant un point de fusion de 55 °C ou plus en une quantité de 0,05 à 1,5 % en masse, dans lequel la cire est sélectionnée parmi le groupe consistant en une cire à base d'hydrocarbures et une cire à base de silicone, et dans lequel la cire à base de silicone est sélectionnée parmi le groupe consistant en une cire d'alkylméthicone, une cire d'alkyldiméthicone et une cire de résine de silsesquioxane ;
   (B) une résine de silicone à base de (méth)acrylique sélectionnée parmi le groupe consistant en un polymère à base de (méth)acrylique ayant une structure de siloxane de type dendrimère à une chaîne latérale et un copolymère de greffon à base de (méth)acrylique-silicone ; et
   (C) un agent de blocage UV en une quantité de 2,5 à 45 % en masse, dans lequel l'agent de blocage UV est sélectionné parmi le groupe consistant en un agent de diffusion UV hydrophobisé et un absorbeur UV, dans lequel l'agent de diffusion UV hydrophobisé est un oxyde métallique particulaire hydrophobisé et l'absorbeur UV est un absorbeur UV organique sélectionné parmi le groupe consistant en un absorbeur UV à base d'acide benzoïque, un absorbeur UV à base d'acide anthranilique, un absorbeur UV à base d'acide salicylique, un absorbeur UV à base d'acide cinnamique, un absorbeur UV à base de benzophénone et un absorbeur UV à base de triazine.

2. Produit cosmétique d'écran solaire selon la revendication 1, dans lequel une teneur du composant (B) est de 0,01 % en masse à 10 % en masse par rapport au produit cosmétique d'écran solaire.

3. Produit cosmétique d'écran solaire selon la revendication 1 ou la revendication 2, dans lequel une teneur du composant (B) est de 0,1 % en masse à 3 % en masse par rapport au produit cosmétique d'écran solaire.

4. Produit cosmétique selon l'une quelconque des revendications 1 à 3, comprenant en outre (D) un agent filmogène à base de silicone qui est un silicone modifié par poly(N-acylalkylèneimine).

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2018221174 A1
- JP 2009084171 A
- JP 2009235007 A
- WO 2019230274 A1
- JP 2017066140 A
- JP 2019014708 A
- JP HEI111530 A
- JP 2000063225 A
- JP HEI225411 A
- JP HEI2132141 A
- JP HEI3162442 A
- JP 2003104825 A
- JP 3187440 B
- JP 2007326902 A
- JP 2212579 A
- JP 3220153 A
- JP 2008143820 A
- JP 2009024114 A
- JP 2015067603 A
- JP 2015168663 A


### Non-patent literature cited in the description


- Database, 1380759